# EUROPEAN PATENT SPECIFICATION

(11) **EP 3 302 516 B1**
(45) Date of publication and mention of the grant of the patent: **30.12.2020**
(21) Application number: 16804090.5
(22) Date of filing: 27.05.2016
(51) Int. Cl.: C07K 5/107, C07K 5/103, A61K 47/54, A61K 47/60, A61K 47/64, A61K 47/65

(54) **BIOLOGICALLY CLEAVABLE TETRAPEPTIDE LINKING AGENTS**
BIOLOGISCH SPALTBARE TETRAPEPTID-LINKER
AGENTS DE LIAISON TÉTRAPEPTIDIQUES BIOLOGIQUEMENT CLIVABLES

(30) Priority: 29.05.2015 US 201562168244 P; 01.10.2015 US 201562235833 P
(43) Date of publication of application: 11.04.2018
(73) Proprietor: Arrowhead Pharmaceuticals, Inc., Pasadena, CA 91105 (US)
(72) Inventor: CARLSON, Jeffrey, C., Madison, WI 53719 (US); BLOKHIN, Andrei, V., Fitchburg, WI 53711 (US)
(74) Representative: van Kooij, Adriaan
(86) International application number: PCT/US2016/034517
(87) International publication number: WO 2016/196243

(56) References cited:
- WO-A1-00/47608
- WO-A1-02/15700
- WO-A1-2009/117531
- WO-A1-2011/131314
- WO-A1-2012/138294
- WO-A1-2015/038426
- US-A1- 2008 214 648
- US-B1- 6 214 345
- US-B1- 7 589 170
- ROZEMA DAVID B ET AL: "Protease-triggered siRNA delivery vehicles", JOURNAL OF CONTROLLED RELEASE, vol. 209, 14 April 2015 (2015-04-14), pages 57-66, XP029173946, ISSN: 0168-3659, DOI: 10.1016/J.JCONREL.2015.04.012
- ARPAN SATSANGI ET AL: "Design of a Paclitaxel Prodrug Conjugate for Active Targeting of an Enzyme Upregulated in Breast Cancer Cells", MOLECULAR PHARMACEUTICS, vol. 11, no. 6, 2 June 2014 (2014-06-02), pages 1906-1918, XP055568961, US ISSN: 1543-8384, DOI: 10.1021/mp500128k

## Description

Physiologically labile linkers or modifiers are useful for a variety of process, including therapeutic drug delivery. The utility of the linker or modifier may be further enhanced if cleavage of the linker regenerates at least one of the original components in an unmodified state without any vestige of the linker or modifier.

Several strategies have been investigated in clinical and preclinical settings to reversibly link or modify a compound. Such reversible conjugates are used to lessen toxic effects and improve pharmacological properties of the compound. To be effective, the reversible conjugate must remain stable in the bloodstream, yet allow for release of the compound after interaction of the conjugate with the target cell. Further, the cleavage of the linker or modifier must be such that the compound be allowed to reach its biochemical target and to interact effectively with it. Often, the compound must be released in an unmodified state.

Examples of reversible conjugates include prodrugs, derivatives of drugs which remain inactive in their prototype form but are metabolized in the body to generate the active drugs, and carriers such as antibody-drug conjugates. The formation of reversible conjugates has been shown to be useful in the development of antitumor chemotherapeutic drugs and in nucleotide delivery.

Rozema et al. (US Patent 8137695) have shown reversible modification of polyamines using dimethylmaleic anhydrides which form pH sensitive maleamide linkages. Delivery of the modified polymer to cells and internalization results in cleavage of the maleamide linkage in the reduced pH environment of endosomes to regenerate polymer amines.

In addition to pH sensitive linkages, peptide-containing linkages have been developed that are activated by proteases in vivo. Rozema et al. (US Patent 8426554) provided a means to reversibly regulate membrane disruptive activity of membrane active polyamines using steric stabilizers or targeting groups conjugated to polymeric amino-containing sidechains via a dipeptide **p**-amidobenzyl-carbamate spacer (PABC). According to the published design, in presence of proteolytic enzymes, hydrolysis of the anilide bond triggers a 1,6-elimination cascade that results in generation of a unmodified polycationic polymer with restored membranolytic properties.

Application of the self-immolative PABC spacer in prodrug design was originally proposed by Carl et al. (1981). This strategy combines cleavage of covalent anilide bond with spontaneous release of the desired substrate. PABC spacers have been extensively studied for controlled drug release of a therapeutic agent, particularly for anticancer therapy (Dorywalska et al. 2015, Zhang et al. 2014, Florent et al 1998, Toki et al. 2002, Shamis et al. 2004, Amir et al. 2005, Amir et al. 2005, Gopin et al. 2006, Zhang et al. 2013, Zhang et al. 2013), However, there is concern that the aza-quinone methide, also known as a quinonimine methide (QIM), generated during PABC elimination, can be a source of toxicity due to its propensity to react with N, O, and S-nucleophiles (Reboud-Ravaux et al. 2009).

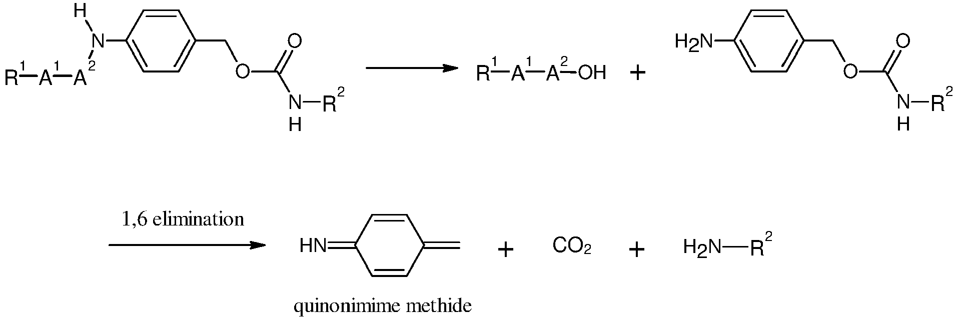

Certain peptide proteolyzable pro-drugs lacking the PABC spacer have been described (Zhong et al. 2013, Cho KY et al. 2012). However, a limitation of the prior described peptide proteolyzable pro-drugs is that they do not liberate the primary amine constituent of the parent drug in a rate comparable to that of self-immolating PABC type analogues. Cleavage of the C-terminal amino acid residue by endopeptidases during proteolysis of the prodrug appears to be the rate limiting step (Masquelier et al. 1980, Schmid et al. 2007, Schmid et al. 2007, Elsadek et al. 2010, Trouet et al. 1982).

WO 02/15700 discloses a modified form of a therapeutic agent and comprises a therapeutic agent, an oligopeptide having a plasmin peptide substrate of 2-4 amino acids and mono- or di-peptide linkage, a stabilizing group and, optionally, a linker group. The prodrug is cleavable by plasmin. Also disclosed are methods of making and using the prodrug compounds.

### SUMMARY

Described herein are tetrapeptide linkers for reversibly linking a first compound to a amine-containing second compound as outlined in the appended claims.

The physiologically cleavable linkers (linkers) comprise a tetrapeptide having the general from: -A⁴-A³-A²-A¹- wherein A⁴ is L-phenylalanine, A³ is L-citruline, A² is L-phenylalanine, and A¹ is L-proline. The tetrapeptide linkers are cleaved (digested) by proteolytic enzymes such as endogenous proteases present in an organism, particularly a mammal, tissue, cell, or subcellular compartment or organelle. Further, proteolytic enzymes readily and rapidly cleave the peptide bond on the C-terminal side of A¹ to liberate the amine-containing second compound. Further described are compositions containing the described tetrapeptide linkers and methods for using the tetrapeptides for reversibly linking two moieties or reversibly modifying an amine or amine-containing compound. The described reversible linkers and modifiers provide for serum stable modification and can be cleaved *in vivo.*

Described herein are tetrapeptide linking agents comprising: R⁵-A⁴-A³-A²-A¹-R⁷, wherein R⁵ comprises a first compound, A⁴ is L-phenylalanine, A³ is L-citruline, A² is L-phenylalanine, A¹ is L-proline, and R⁷ is an amine-reactive group. R⁷ is chosen such that reaction with an amine or amine-containing compound forms an amide bond with A¹. A³ can be a polar uncharged L-amino acid. The tetrapeptide linking agents can be used to reversibly link the first compound to the amine-containing compound. The tetrapeptide linking agents can be used to reversibly modify an amine-containing compound. The tetrapeptide linking agents can be used to reversibly modify a polyamine such as a membrane active polyamine.

We describe compositions comprising a first compound linked to a second compound via a tetrapeptide linking agent wherein the tetrapeptide linking agent consists of A⁴A³A²A¹ wherein A⁴ is L-phenylalanine, A³ is L-citruline, A² is L-phenylalanine, and A¹ is L-proline, and A¹ is linked to the second compound via an amide bond. Cleavage (digestion) of the tetrapeptide by proteolytic enzymes *in vitro* or *in vivo* results in cleavage between A¹ and the second compound to liberate the second compound. Disclosed is A⁴A³A²A¹ having the sequence: FCitFP (SEQ ID NO: 12), VCitFP (SEQ ID NO: 19), ACitFP (SEQ ID NO: 3), FKFP (SEQ ID NO: 16), FCitVP (SEQ ID NO: 13), FCitFL (SEQ ID NO: 11), FCitF(Nme)A (SEQ ID NO: 9), or FCitAP (SEQ ID NO: 8), wherein F is L-phenylalanine, Cit is L-citrulline, P is L-proline, V is L-Valine, A is L-alanine, K is L-Lysine, L is L-leucine, (Nme)A is L-N-methyl-alanine.

Compositions comprising reversibly modified polyamines are described. The polyamines are modified by reversible modification of a plurality of amines on the polyamine with the herein described tetrapeptide linking agents. The polyamine can be an amphipathic membrane active polyamine. The composition can further comprise an RNAi trigger. The polyamine can be covalently linked to the RNAi trigger. The linkage for covalent attachment of the polyamine to the RNAi trigger can contain a physiologically labile linkage such as a disulfide bond. The polyamine can be not covalently linked to the RNAi trigger and the RNAi trigger can be covalently linked to a targeting group. The composition further comprises a pharmaceutically acceptable excipient.

Methods for linking a first compound to a amine-containing second compound are described, the methods comprising: attaching the first compound to the amino-terminus of a tetrapeptide and forming an amide bond between the carboxy-terminus of the tetrapeptide and the amine-containing second compound, wherein the tetrapeptide has the amino acid sequence A⁴A³A²A¹, wherein A⁴ is L-phenylalanine, A³ is L-citrulline,. A² is L-phenylalanine and A¹ is L-proline.

Disclosed are the tetrapeptide linker or linking agent having the four amino acid sequence selected from the group consisting of: FCitFP (SEQ ID NO: 12), VCitFP (SEQ ID NO: 19), ACitFP (SEQ ID NO: 3), FKFP (SEQ ID NO: 16), FCitVP (SEQ ID NO: 13), FCitFL (SEQ ID NO: 11), FCitF(Nme)A (SEQ ID NO: 9), FCitAP (SEQ ID NO: 8), wherein F is L-phenylalanine, Cit is L-citrulline, P is L-proline, V is L-Valine, A is L-alanine, K is L-Lysine, L is L-leucine, (Nme)A is L-N-methyl-alanine.

Described herein is the use of Phenylalanine-Citrulline-Phenylalanine-Proline (FCitFP (SEQ ID NO: 12)) tetrapeptide to form a physiologically cleavable linker. The FCitFP linker is rapidly cleaved by proteolytic enzymes in a mammal, mammalian tissue, mammalian cell, or mammalian subcellular compartment or organelle. In some embodiments, an amine-containing compound is linked to the proline via an amide bond. The proline C-terminal amide bond is efficiently cleaved by proteases *in vivo* to release the amine-containing compound.

In some embodiments, the first compound can be selected from the group comprising: targeting group, cell receptor ligand, integrin ligand, RGD ligand, RGD mimic, asialoglycoprotein receptor (ASGPr) ligand, galactose, galactose derivative, N-acetylgalactosamine, folate, steric stabilizer, polyethylene glycol (PEG), polynucleotide, polymer, polyamine, antibody, drug product, hapten, digoxigenin, vitamin, biotin, fluorophore, antibody, monoclonal antibody, and antibody fragment.

In some embodiments, the amine-containing second compound can be selected from the group comprising: targeting group, cell receptor ligand, integrin ligand, RGD ligand, RGD mimic, asialoglycoprotein receptor (ASGPr) ligand, galactose, galactose derivative, N-acetylgalactosamine, folate, steric stabilizer, polyethylene glycol (PEG), polynucleotide, polymer, polyamine, antibody, drug product, hapten, digoxigenin, vitamin, biotin, antibody, monoclonal antibody, and antibody fragment.

In some embodiments, compositions for delivering an RNAi trigger to a cell *in vivo* comprising are described.

### BRIEF DESCRIPTION OF THE FIGURES

- FIG. 1.: Structures for some components used in preparation or testing of the described tetrapeptide linkers.
- FIG. 2.: Graph illustrating: A) Rate of digestion for compound **39,** and B) Rate of digestion for compound **29.**

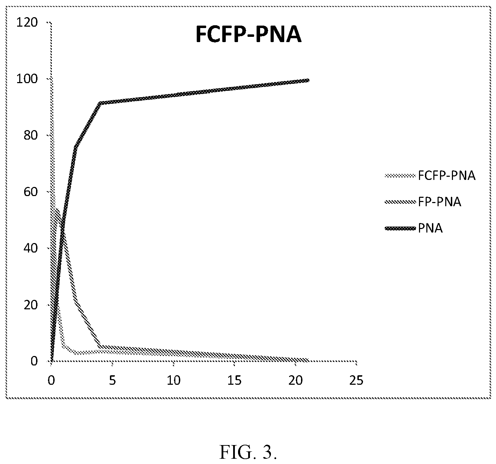

- FIG. 3.: Graph illustrating reaction kinetics for compounds **29** and **39,** rates of intermediates generated (%) over time (hr) (C = citrulline).

### DETAILED DESCRIPTION

Described are tetrapeptide linkers and tetrapeptide linking agents useful for reversibly linking a first compound with an amine-containing second compound and/or reversibly modifying the amine-containing second compound. The described tetrapeptide linkers exhibit improved kinetics of cleavage over previously described peptide linkers used for prodrug delivery (Rejmanova et al. 1983, Malugin et al. 2007, Miller et al. 2009, Soler et al. 2015, Chu et al. 2012). The tetrapeptide linkers are stable to hydrolysis in absence of proteolytic enzymes. In the presence of proteolytic enzymes (also called proteinases, proteases, or peptidases) *in vitro* or in *vivo,* the tetrapeptide is readily cleaved. More specifically, the described tetrapeptide linkers and tetrapeptide linking agents are rapidly cleaved between the carboxy terminal amino acid of the tetrapeptide and the amine-containing second compound, releasing the amine-containing second compound.

Tetrapeptide linkers are described comprising the structure represented by: 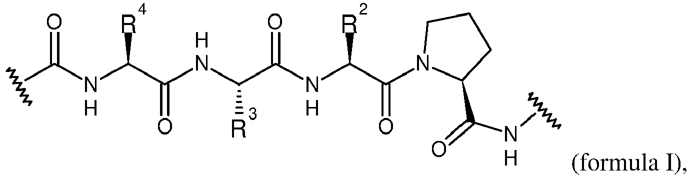 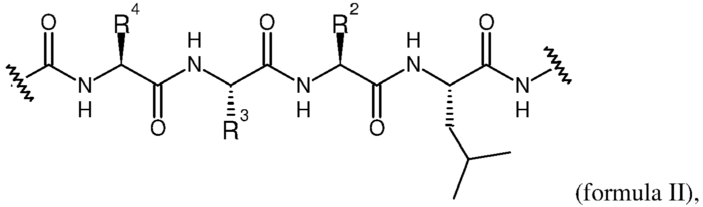 or 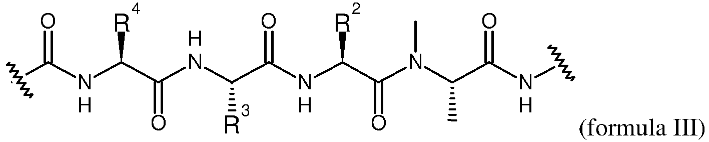 wherein,
R⁴ is an R-group (side chain) of a natural, non-natural isomeric, or synthetic hydrophobic L amino acid wherein the hydrophobicity index (Monera et al, J. Protein Sci. 1995, 1, 319) at pH 7 is 41 or greater, normalized to glycine, as it relates to the composition of the amino acid side chain (R-group),
R³ is an R-group (side chain) of a uncharged hydrophilic or basic hydrophilic L amino acid wherein the hydrophobicity index (Monera et al, J. Protein Sci. 1995, 1, 319) at pH 7 is -28 or less, normalized to glycine, as it relates to the composition of amino acid side chain (R-group), and
R² is an R group (side chain) of a natural, non-natural isomeric, or synthetic hydrophobic L amino acid wherein the hydrophobicity index (Monera et al, J. Protein Sci. 1995, 1, 319) at pH 7 is 41 or greater, normalized to glycine, as it relates to the composition of the amino acid side chain (R-group).
R² and R⁴ can be independently selected from the group consisting of: -CH₃, -CH(CH₃)₂, -CH₂CH(CH₃)₂, -CH(CH₃)CH₂CH₃, and -CH₂C₆H₆ (phenylalanine).
R³ can be selected from the group consisting of: -(CH₂)₃NHC(=O)NH₂, -CH₂CONH₂, -(CH₂)₄NH₃⁺, and -(CH₂)₃-NH-C(=NH)-NH₂.

Following exposure to proteases *in vivo,* such as in a lysosome, the tetrapeptide C-terminal amide bond is rapidly cleaved (digested).

Tetrapeptide linkers according to the present invention comprise the structure represented by:

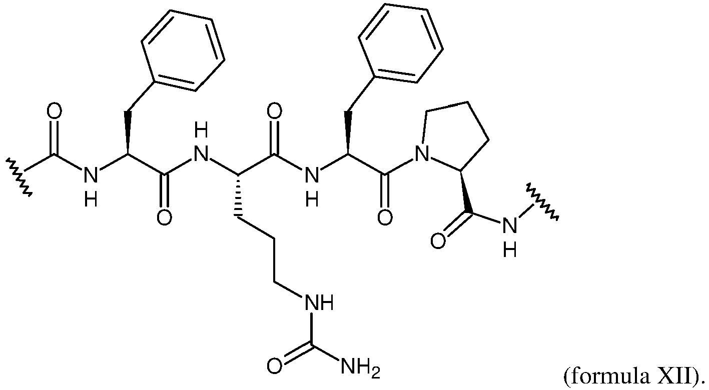

In some embodiments, biologically labile compounds are described, wherein the biologically labile compounds include a first compound linked to an amine-containing second compound via a tetrapeptide linker comprising:

R⁵-A⁴-A³-A²-A¹-R⁶ (formula IV)

wherein
R⁵ represents the first compound,
R⁶ represents the amine-containing second compound,
A⁴ is L-phenylalanine;
A³ is L-citrulline;
A² is L-phenylalanine;
A¹ is L-proline, and
A¹ is linked to R⁶ via an amide bond.

A¹ is L-proline, A² and A⁴ are L-phenylalanine, and A³ is L-citrulline (FCitFP (SEQ ID NO: 12)).

Disclosed is A¹ is L-proline, A² is L-phenylalanine, A³ is L-lysine, and A⁴ is L-phenylalanine (FKFP (SEQ ID NO: 16)).

Disclosed is A¹ is L-proline, A² is L-valine, A³ is L-citrulline, and A⁴ is L-phenylalanine (FCitVP (SEQ ID NO: 13)).

Disclosed is A¹ is L-proline, A² is L-phenylalanine, A³ is L-citrulline, and A⁴ is L-valine (VCitFP (SEQ ID NO: 19)).

Disclosed is A¹ is L-proline, A² is L-alanine, A³ is L-citrulline, and A⁴ is L-phenylalanine (FCitAP (SEQ ID NO: 8)).

Disclosed is A¹ is L-proline, A² is L-phenylalanine, A³ is L-citrulline, and A⁴ is L-alanine (ACitFP (SEQ ID NO: 3)).

Disclosed is A¹ is leucine, A² and A⁴ are independently L-alanine, L-valine, L-leucine, L-isoleucine or L-phenylalanine (side chains of -CH₃, -CHCH₃, -CH(CH₃)₂, -CH₂CH(CH₃)₂, -CH(CH₃)CH₂CH₃, or -CH₂C₆H₆, respectively), and A³ is L-citrulline, L-asparagine, or L-lysine, or L-arginine (side chains of -(CH₂)₃NHCONH₂, -CH₂CONH₂, or -(CH₂)₄NH₃⁺, respectively).

Disclosed is A¹ is L-leucine, A² is L-phenylalanine, A³ is L-citrulline, and A⁴ is L-phenylalanine (FCitFL (SEQ ID NO: 11)).

Disclosed is A¹ is L-N-methyl-alanine, A² and A⁴ are independently L-alanine, L-valine, L-leucine, L-isoleucine or L-phenylalanine (side chains of -CH₃, -CHCH₃, -CH(CH₃)₂, -CH₂CH(CH₃)₂, -CH(CH₃)CH₂CH₃, or -CH₂C₆H₆, respectively), and A³ is L-citrulline, L-asparagine, L-lysine, L-arginine (side chains of -(CH₂)₃NHCONH₂, -CH₂CONH₂, or -(CH₂)₄NH₃⁺, or -(CH₂)₄NH₃⁺, respectively).

Disclosed is A¹ is L-N-methyl-alanine, A² is L-phenylalanine, A³ is L-citrulline, and A⁴ is L-phenylalanine (FCitF(Nme)A (SEQ ID NO: 9)).

In some embodiments, R₅ comprises a compound selected from the list comprising steric stabilizer, PEG, H-(CH₂)₀₋₂-(O-CH₂-CH₂)₁₋₅₀₀-O₀₋₁-(CH₂)₀₋₂-, PEG₁₋₁₀₀, targeting group, cell receptor ligand, integrin-binding ligand, RGD ligand, RGD mimic, asialoglycoprotein receptor (ASGPr) ligand, galactose, galactose derivative, N-acetylgalactosamine, folate, polynucleotide, polymer, polyamine, antibody, drug product, hapten, digoxigenin, vitamin, biotin, fluorophore, antibody, immunoglobulin, monoclonal antibody, and antibody fragment.

R₆ is a primary amine-containing compounds selected from the group comprising: steric stabilizer, PEG, H-(CH₂)₀₋₂-(O-CH₂-CH₂)₁₋₅₀₀-O₀₋₁-(CH₂)₀₋₂-, PEG₁₋₁₀₀, targeting group, cell receptor ligand, integrin-binding ligand, RGD ligand, RGD mimic, asialoglycoprotein receptor (ASGPr) ligand, galactose, galactose derivative, N-acetylgalactosamine, folate, polynucleotide, amine-modified polynucleotide, polymer, amine-containing polymer, polyamine, amphipathic membrane active polyamine, antibody, drug product, hapten, digoxigenin, vitamin, biotin, fluorophore, antibody, monoclonal antibody, and antibody fragment.

Disclosed are tetrapeptide modifying agents for reversibly attaching a first compound to an amine-containing second compound via a biologically labile tetrapeptide linker are described, the tetrapeptide modifying agents comprising:

R⁵-A⁴-A³-A²-A¹-R⁷ (formula V)

wherein
R⁵ represents the first compound,
A⁴ is a natural, non-natural isomeric, or synthetic hydrophobic L amino acid wherein the hydrophobicity index (Monera et al, J. Protein Sci. 1995, 1, 319) at pH 7 is 41 or greater, normalized to glycine, as it relates to the composition of the amino acid side chain (R-group),
A³ is an uncharged or basic hydrophilic L amino acid wherein the hydrophobicity index (Monera et al, J. Protein Sci. 1995, 1, 319) at pH 7 is -28 or less, normalized to glycine, as it relates to the composition of amino acid side chain (R-group),
A² is a natural, non-natural isomeric, or synthetic hydrophobic L amino acid wherein the hydrophobicity index (Monera et al, J. Protein Sci. 1995, 1, 319) at pH 7 is 41 or greater, normalized to glycine, as it relates to the composition of the amino acid side chain (R-group),
A¹ is L-proline, L-leucine, or L-N-methyl alanine, and
R⁷ comprises an amine-reactive group such as but not limited to: activated esters of TFP (tetrafluoro phenyl) or NHS (N-hydroxysuccinimide) 2-MT (2-mercaptothiazoline).

Disclosed is A¹ is proline, A² and A⁴ are independently alanine, valine, leucine, isoleucine or phenylalanine (side chains of -CH₃, -CH(CH₃)₂, -CH₂CH(CH₃)₂, -CH(CH₃)CH₂CH₃, or -CH₂C₆H₆, respectively), and A³ is citrulline or asparagine (side chains of -(CH₂)₃NHCONH₂ or -CH₂CONH₂, respectively).

Disclosed is A¹ is proline, A² and A⁴ are phenylalanine, and A³ is citrulline (FCitFP (SEQ ID NO: 12)). Disclosed is A¹ is proline, A² is phenylalanine, A³ is citrulline, and A⁴ is alanine (ACitFP (SEQ ID NO: 3)).

Disclosed is A¹ is L-proline, A² and A⁴ are independently L-alanine, L-valine, L-leucine, L-isoleucine or L-phenylalanine (side chains of -CH₃, -CHCH₃, -CH(CH₃)₂, -CH₂CH(CH₃)₂, -CH(CH₃)CH₂CH₃, or -CH₂C₆H₆, respectively), and A³ is L-citrulline, L-asparagine, or L-lysine (side chains of -(CH₂)₃NHCONH₂, -CH₂CONH₂, or -(CH₂)₄NH₃⁺, respectively).

Disclosed is A¹ is L-proline, A² and A⁴ are L-phenylalanine, and A³ is L-citrulline (FCitFP (SEQ ID NO: 12)).

Disclosed is A¹ is L-proline, A² is L-phenylalanine, A³ is L-lysine, and A⁴ is L-phenylalanine (FKFP (SEQ ID NO: 16)).

Disclosed is A¹ is L-proline, A² is L-valine, A³ is L-citrulline, and A⁴ is L-phenylalanine (FCitVP (SEQ ID NO: 13)).

Disclosed is A¹ is L-proline, A² is L-phenylalanine, A³ is L-citrulline, and A⁴ is L-valine (VCitFP (SEQ ID NO: 19)).

Disclosed is A¹ is L-proline, A² is L-alanine, A³ is L-citrulline, and A⁴ is L-phenylalanine (FCitAP (SEQ ID NO: 8)).

Disclosed is A¹ is L-proline, A² is L-phenylalanine, A³ is L-citrulline, and A⁴ is L-alanine (ACitFP (SEQ ID NO: 3)).

Disclosed is A¹ is leucine, A² and A⁴ are independently L-alanine, L-valine, L-leucine, L-isoleucine or L-phenylalanine (side chains of -CH₃, -CHCH₃, -CH(CH₃)₂, -CH₂CH(CH₃)₂, -CH(CH₃)CH₂CH₃, or -CH₂C₆H₆, respectively), and A³ is L-citrulline, L-asparagine, or L-lysine (side chains or -(CH₂)₃NHCONH₂, -CH₂CONH₂, of -(CH₂)₄NH₃⁺, respectively).

Disclosed is A¹ is L-leucine, A² is L-phenylalanine, and A³ is L-lysine, and A⁴ is L-phenylalanine (FKFL (SEQ ID NO: 15)).

Disclosed is A¹ is L-leucine, A² is L-phenylalanine, A³ is L-citrulline, and A⁴ is L-phenylalanine (FCitFL (SEQ ID NO: 11)).

Disclosed is A¹ is L-N-methyl-alanine, A² and A⁴ are independently L-alanine, L-valine, L-leucine, L-isoleucine or L-phenylalanine (side chains of -CH₃, -CHCH₃, -CH(CH₃)₂, -CH₂CH(CH₃)₂, -CH(CH₃)CH₂CH₃, or -CH₂C₆H₆, respectively), and A³ is L-citrulline, L-asparagine, or L-lysine (side chains of -(CH₂)₃NHCONH₂, -CH₂CONH₂, or -(CH₂)₄NH₃⁺, respectively).

Disclosed is A¹ is L-N-methyl-alanine, A² is L-phenylalanine, and A³ is L-citrulline, and A⁴ is L-phenylalanine (FCitF(Nme)A (SEQ ID NO: 9)).

In some embodiments, R₅ comprises a compound selected from the list comprising steric stabilizer, PEG, H-(CH₂)₀₋₂-(O-CH₂-CH₂)₁₋₅₀₀-O₀₋₁-(CH₂)₀₋₂-, PEG₁₋₁₀₀, targeting group, cell receptor ligand, integrin-binding ligand, RGD ligand, RGD mimic, asialoglycoprotein receptor (ASGPr) ligand, galactose, galactose derivative, N-acetylgalactosamine, folate, polynucleotide, polymer, polyamine, lipid, liposome, antibody, drug product, hapten, digoxigenin, vitamin, biotin, fluorophore, antibody, immunoglobulin, monoclonal antibody, and antibody fragment.

In some embodiments, a targeting group comprises a cell surface receptor ligand. A cell surface receptor can include, but is not limited to, asialoglycoprotein receptor and integrin receptor. An asialoglycoprotein receptor ligand can include, but is not limited to, galactose, galactosamine, N-formylgalactosamine, N-acetylgalactosamine, N-propionyl-galactosamine, N-n-butanoylgalactosamine, and N-iso-butanoylgalactosamine, or dimer, trimers or tetramers thereof. An integrin receptor can include, but is not limited to, an αvβ3 integrin and an αvβ6 integrin. An integrin receptor ligand can include, but is not limited to, an RGD or RGD mimic (see for example, US Patent Publication US-2015-0045573 A1).

A steric stabilizer can include, but is not limited to, a polyethylene glycol (PEG). A PEG contains 1-120 ethylene units, 3-30 ethylene units or 3-24 ethylene units.

Disclosed are compounds for reversibly attaching a first compound to an amine-containing second compound via a biologically labile tetrapeptide linker are described, the compounds comprising the structure represented by: 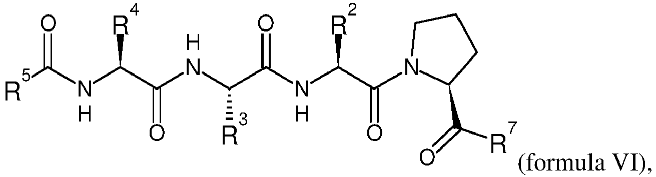 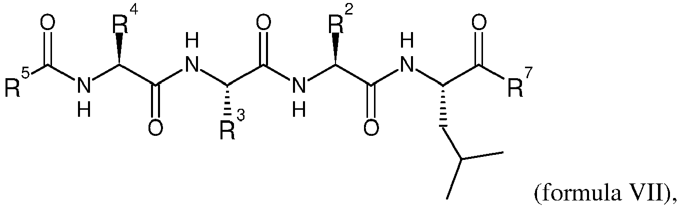 or 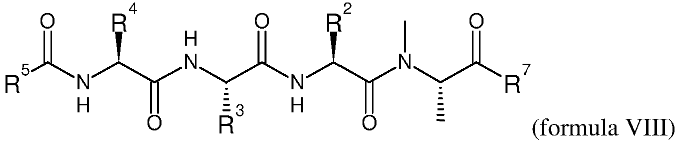 wherein
R⁵ represents the first compound,
R⁴ is an R-group (side chain) of a natural, non-natural isomeric, or synthetic hydrophobic L amino acid wherein the hydrophobicity index (Monera et al, J. Protein Sci. 1995, 1, 319) at pH 7 is 41 or greater, normalized to glycine, as it relates to the composition of the amino acid side chain (R-group)
R³ is an R-group (side chain) of a uncharged hydrophilic or basic hydrophilic L amino acid wherein the hydrophobicity index (Monera et al, J. Protein Sci. 1995, 1, 319) at pH 7 is -28 or less, normalized to glycine, as it relates to the composition of amino acid side chain (R-group), and
R² is an R group (side chain) of a natural, non-natural isomeric, or synthetic hydrophobic L amino acid wherein the hydrophobicity index (Monera et al, J. Protein Sci. 1995, 1, 319) at pH 7 is 41 or greater, normalized to glycine, as it relates to the composition of the amino acid side chain (R-group).
R⁷ comprises an amine-reactive group.

R⁷ is chosen such that reaction with an amine or amine-containing compound forms an amide bond with A¹. In some embodiments, amine-reactive group is TFP (tetrafluoro phenyl), or NHS (N-hydroxysuccinimide).

Disclosed is R² and R⁴ are independently selected from the group consisting of:
-CH₃, -CH(CH₃)₂, -CH₂CH(CH₃)₂, -CH(CH₃)CH₂CH₃, and -CH₂C₆H₆.

Disclosed is R³ is selected from the group consisting of: -(CH₂)₃NHCONH₂ and -CH₂CONH₂.

Disclosed are compounds for reversibly attaching a first compound to an amine-containing second compound via a biologically labile tetrapeptide linker are described, the compounds comprising the structure represented by: 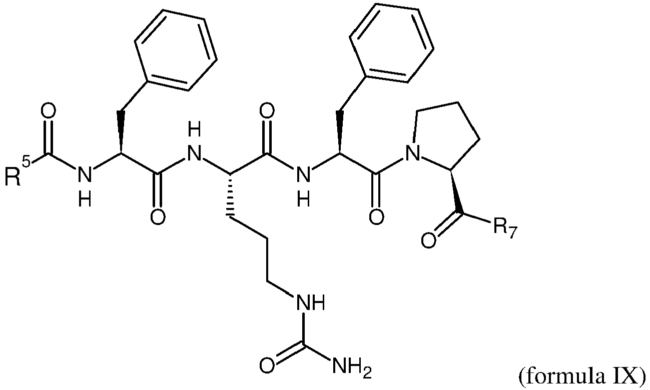 wherein R⁵ represents the first compound and R⁷ comprises an amine reactive group. R⁷ is chosen such that reaction with an amine or amine-containing compound forms an amide bond with A¹. In some embodiments, the amine-reactive group is TFP (tetrafluoro phenyl), or NHS (N-hydroxysuccinimide).

Reversibly modified polyamines are described, a reversibly modified polyamine comprising:

(R⁵-A⁴-A³-A²-A¹)ₙ-P (formula X)

wherein
R⁵, A⁴, A³, A², and A¹ are each as described above,
P comprises a polyamine,
n is an integer greater than or equal to 1, and
each A¹ is linked to an amine on the polyamine via an amide bond.

The polyamine can be a membrane active polyamine such as an amphipathic membrane active polyamine.

Disclosed is A¹ is L-proline, A² and A⁴ are independently L-alanine, L-valine, L-leucine, L-isoleucine or L-phenylalanine (side chains of -CH₃, -CHCH₃, -CH(CH₃)₂, -CH₂CH(CH₃)₂, -CH(CH₃)CH₂CH₃, or -CH₂C₆H₆, respectively), and A³ is L-citrulline, L-asparagine, or L-lysine (side chains of -(CH₂)₃NHCONH₂, -CH₂CONH₂, or -(CH₂)₄NH₃⁺, respectively).

According to the invention, A¹ is L-proline, A² and A⁴ are L-phenylalanine, and A³ is L-citrulline (FCitFP (SEQ ID NO: 12)).

Disclosed is A¹ is L-proline, A² is L-phenylalanine, A³ is L-lysine, and A⁴ is L-phenylalanine (FKFP (SEQ ID NO: 16)).

Disclosed is A¹ is L-proline, A² is L-valine, A³ is L-citrulline, and A⁴ is L-phenylalanine (FCitVP (SEQ ID NO: 13)).

Disclosed is A¹ is L-proline, A² is L-phenylalanine, A³ is L-citrulline, and A⁴ is L-valine (VCitFP (SEQ ID NO: 19)).

Disclosed is A¹ is L-proline, A² is L-alanine, A³ is L-citrulline, and A⁴ is L-phenylalanine (FCitAP (SEQ ID NO: 8)).

Disclosed is A¹ is L-proline, A² is L-phenylalanine, A³ is L-citrulline, and A⁴ is L-alanine (ACitFP (SEQ ID NO: 3)).

Disclosed is A¹ is leucine, A² and A⁴ are independently L-alanine, L-valine, L-leucine, L-isoleucine or L-phenylalanine (side chains of -CH₃, -CHCH₃, -CH(CH₃)₂, -CH₂CH(CH₃)₂, -CH(CH₃)CH₂CH₃, or -CH₂C₆H₆, respectively), and A³ is L-citrulline, L-asparagine, or L-lysine (side chains of -(CH₂)₃NHCONH₂, -CH₂CONH₂, or -(CH₂)₄NH₃⁺, respectively).

Disclosed is A¹ is L-leucine, A² is L-phenylalanine, A³ is L-lysine, and A⁴ is L-phenylalanine (FKFL (SEQ ID NO: 15)).

Disclosed is A¹ is L-leucine, A² is L-phenylalanine, A³ is L-citrulline, and A⁴ is L-phenylalanine (FCitFL (SEQ ID NO: 11)).

Disclosed is A¹ is L-N-methyl-alanine, A² and A⁴ are independently L-alanine, L-valine, L-leucine, L-isoleucine or L-phenylalanine (side chains of -CH₃, -CHCH₃, -CH(CH₃)₂, -CH₂CH(CH₃)₂, -CH(CH₃)CH₂CH₃, or -CH₂C₆H₆, respectively), and A³ is L-citrulline, L-asparagine, or L-lysine (side chains of -(CH₂)₃NHCONH₂, -CH₂CONH₂, or -(CH₂)₄NH₃⁺, respectively).

Disclosed is A¹ is L-N-methyl-alanine, A² is L-phenylalanine, and A³ is L-citrulline, and A⁴ is L-phenylalanine (FCitF(Nme)A (SEQ ID NO: 9)).

In some embodiments, the reversibly modified polyamine is formed by reacting the polyamine with a plurality of described tetrapeptide modifying agents. In some embodiments, attachment of the tetrapeptide modifying agents to a membrane active polyamine masks membrane activity of the membrane active polyamine by forming a masked polyamine or delivery polymer.

In some embodiments, greater the 50% of the amines on the polyamine are modified (the value of n is greater than 50% of the number of amines on the polyamine). In some embodiments, greater the 60% of the amines on the polyamine are modified. In some embodiments, greater the 70% of the amines on the polyamine are modified. In some embodiments, greater the 75% of the amines on the polyamine are modified. In some embodiments, greater the 80% of the amines on the polyamine are modified. In some embodiments, greater the 85% of the amines on the polyamine are modified. In some embodiments, greater the 90% of the amines on the polyamine are modified. In some embodiments, greater the 95% of the amines on the polyamine are modified. In some embodiments, 100% of the amines on the polyamine are modified.

In some embodiments, R⁵ comprises a targeting group and the reversibly modified polyamine is further conjugated to an RNAi trigger. The RNAi trigger-reversibly modified polyamine conjugate can be used to deliver the RNAi trigger to cells in vivo for the purpose of knocking down target gene expression. The conjugate is formed and administered to a patient. Administration can be, but is not limited, to intravascular injection and subcutaneous injection. Cleavage (digestion) of the tetrapeptides by proteolytic enzymes in vitro or in vivo results in cleavage between A¹ and the polyamine to liberate the polyamine. The rate of release of the polyamine is sufficient to provide drug or RNAi trigger delivery.

Reversibly modified polyamines are described, a reversibly modified polyamine comprising:

(R⁹-A⁴-A³-A²-A¹)ₙ-P-(A^{1'}-A^{2'}-A^{3'}-A^{4'}-R⁸)ₘ (formula XI)

wherein
A⁴, A³, A², and A¹ are each as described above,
A^{1'}, A^{2'}, A^{3'}, and A^{4'} are independently as described above for A¹, A², A³, and A⁴, respectively,
R⁹ comprises a targeting group,
R⁸ comprises a steric stabilizer
P comprises a polyamine,
n and m are each integers greater than or equal to 1, and
each A¹ and A^{1'} are linked to amines on the polyamine via amide bonds.

The reversibly modified polyamine is formed by reacting the polyamine with a plurality of described tetrapeptide modifying agents.

The polyamine is a membrane active polyamine. In some embodiments, the membrane active polyamine is an amphipathic membrane active polyamine.

Greater the 50% of the amines on the polyamine are modified (the value of n+m is greater than 50% of the number of amines on the polyamine). In some embodiments, greater the 60% of the amines on the polyamine are modified. In some embodiments, greater the 70% of the amines on the polyamine are modified. In some embodiments, greater the 75% of the amines on the polyamine are modified. In some embodiments, greater the 80% of the amines on the polyamine are modified. In some embodiments, greater the 85% of the amines on the polyamine are modified. In some embodiments, greater the 90% of the amines on the polyamine are modified. In some embodiments, greater the 95% of the amines on the polyamine are modified. In some embodiments, 100% of the amines on the polyamine are modified.

The reversibly modified polyamine can be further conjugated to an RNAi trigger. The RNAi trigger-reversibly modified polyamine conjugate can be used to deliver the RNAi trigger to cells in vivo for the purpose of knocking down target gene expression. The conjugate is formed and administered to a patient. Administration can be, but is not limited, to intravascular injection and subcutaneous injection. Cleavage (digestion) of the tetrapeptides by proteolytic enzymes *in vitro* or *in vivo* results in cleavage between A¹ and the polyamine to liberate the polyamine. The rate of release of the polyamine is sufficient to provide drug or RNAi trigger delivery.

Surprisingly, we show that insertion of a C-terminal L-proline residue of a tetrapeptide linker greatly improves the cleavage characteristics of the linker. Regeneration of H₂N-R⁶ (H₂N-P from formulae X and XI) occurs at a significantly faster rate when L-proline is present at A¹. Without wishing to be bound be theory, endogenous enzymes appear to rapidly cleave the tetrapeptide between A³ and A² to generate the first compound linked to an A⁴-A³ dipeptide (R⁵-A⁴-A³-CO₂H) and the second compound linked to an A²-A¹ dipeptide (H₂N-A²-A¹-N(H)-R⁶). The presence of proline at A¹ suppresses cleavage between A² and A¹. If several amino acids other than proline is present of position A¹, endogenous exoproteases appear to rapidly cleave A² from (H₂N-A²-A¹-N(H)-R⁶) to yield H₂N-A²-CO₂H plus H₂N-A¹-N(H)-R⁶. Cleavage of the single amino acid from the second compound appears to be slow, resulting in slow regeneration of the original amine-containing compound (H₂N-R⁶). In the absence of proline at position A¹, we observed that single amino acid conjugates (H₂N-A¹-N(H)-R²) were readily formed, either by initial cleavage between A² and A¹ or by cleavage between A³ and A² followed by cleavage between A² and A¹. In contrast, if proline, is present at position A¹, then the dipeptide A²-A¹ appears to be rapidly cleaved by endogenous dipeptidases to yield H₂N-A²-Pro-CO₂H and the released second compound H₂N-R⁶. Similar results are achieved with L-leucine or N-methyl alanine and position A¹.

As used herein, membrane active polyamines are surface active, amphipathic polymers that are able to induce one or more of the following effects upon a biological membrane: an alteration or disruption of the membrane that allows non-membrane permeable molecules to enter a cell or cross the membrane, pore formation in the membrane, fission of membranes, or disruption or dissolving of the membrane. As used herein, a membrane, or cell membrane, comprises a lipid bilayer. The alteration or disruption of the membrane can be functionally defined by the polymer's activity in at least one the following assays: red blood cell lysis (hemolysis), liposome leakage, liposome fusion, cell fusion, cell lysis, and endosomal release. Membrane active polymers that can cause lysis of cell membranes are also termed membrane lytic polymers. Polymers that preferentially cause disruption of endosomes or lysosomes over plasma membrane are considered endosomolytic. The effect of membrane active polymers on a cell membrane may be transient. Membrane active polymers possess affinity for the membrane and cause a denaturation or deformation of bilayer structures. Membrane active polymers may be synthetic or non-natural amphipathic polymers.

Delivery of a polynucleotide to a cell is mediated by the membrane active polymer disrupting or destabilizing the plasma membrane or an internal vesicle membrane (such as an endosome or lysosome), including by forming a pore in the membrane, or disrupting endosomal or lysosomal vesicles thereby permitting release of the contents of the vesicle into the cell cytoplasm.

As used herein, a steric stabilizer is a non-ionic hydrophilic polymer (either natural, synthetic, or non-natural) that prevents or inhibits intramolecular or intermolecular interactions of a molecule to which it is attached relative to the molecule containing no steric stabilizer. A steric stabilizer hinders a molecule to which it is attached from engaging in electrostatic interactions. Electrostatic interaction is the non-covalent association of two or more substances due to attractive forces between positive and negative charges. Steric stabilizers can inhibit interaction with blood components and therefore opsonization, phagocytosis, and uptake by the reticuloendothelial system. Steric stabilizers can thus increase circulation time of molecules to which they are attached. Steric stabilizers can also inhibit aggregation of a molecule. In some embodiments, a steric stabilizer is a polyethylene glycol (PEG) or PEG derivative. Suitable PEG molecules have about 1-120 ethylene glycol monomers.

Targeting groups (also targeting ligands) are used for targeting or delivery of a compound to target cells or tissues, or specific cells types. Targeting groups enhance the association of molecules with a target cell. Thus, targeting groups can enhance the pharmacokinetic or biodistribution properties of a conjugate to which they are attached to improve cellular distribution and cellular uptake of the conjugate. Binding of a targeting group, such as a ligand, to a cell or cell receptor may initiate endocytosis. Targeting groups may be selected from the group comprising: compounds with affinity to cell surface molecule, cell receptor ligands, and antibodies, antibody fragments, and antibody mimics with affinity to cell surface molecules. In some embodiments, the targeting group comprises a cell receptor ligand. A variety of ligands have been used to target drugs and genes to cells and to specific cellular receptors. Cell receptor ligands may be selected from the group comprising: carbohydrates, glycans, saccharides (including, but not limited to: galactose, galactose derivatives, mannose, and mannose derivatives), vitamins, folate, biotin, aptamers, peptides (including, but not limited to: RGD-containing peptides, insulin, EGF, and transferrin), and RGC mimics.

As used herein, a ASGPr ligand (or ASGPr ligand) comprises a galactose and galactose derivative having affinity for the ASGPr equal to or greater than that of galactose. Binding of galactose targeting groups to the ASGPr(s) facilitates cell-specific targeting of the delivery peptide to hepatocytes and endocytosis of the delivery peptide into hepatocytes. ASGPr ligands may be selected from the group comprising: lactose, galactose, N-acetylgalactosamine (GalNAc), galactosamine, N-formylgalactosamine, N-acetylgalactosamine, N-propionylgalactosamine, N-n-butanoylgalactosamine, and N-iso-butanoylgalactosamine (Iobst, S.T. and Drickamer, K. J.B.C. 1996, 271, 6686). ASGPr ligands can be monomeric (e.g., having a single galactosamine) or multimeric (e.g., having multiple galactosamines).

RNAi triggers (also called dsRNAi triggers) inhibit gene expression through the biological process of RNA interference (RNAi). RNAi triggers comprise double stranded RNA or RNA-like structures typically containing 15-50 base pairs or 18-26 base pairs and having a nucleobase sequence at least 90% complementary over a core region to a coding sequence in an expressed target gene within the cell. RNAi triggers include, but are not limited to: short interfering RNAs (siRNAs), double-strand RNAs (dsRNA), micro RNAs (miRNAs), short hairpin RNAs (shRNA), and dicer substrates (US Patent Nos. 8,084,599 8,349,809 and 8,513,207).

By inhibit, down-regulate, or knockdown gene expression, it is meant that the expression of the gene, as measured by the level of mRNA transcribed from the gene or the level of polypeptide, protein or protein subunit translated from the mRNA, is reduced below that observed in the absence of the blocking polynucleotide-conjugates described herein. Inhibition, down-regulation, or knockdown of gene expression, with a polynucleotide delivered by the compositions described herein, is below that level observed in the presence of a control inactive nucleic acid, a nucleic acid with scrambled sequence or with inactivating mismatches, or in absence of conjugation of the polynucleotide to the reversibly modified polymer.

In pharmacology and toxicology, a route of administration is the path by which a drug, fluid, poison, or other substance is brought into contact with the body. In general, methods of administering drugs and nucleic acids for treatment of a mammal are well known in the art and can be applied to administration of the compositions described herein. The compositions described herein can be administered via any suitable route, including parenterally, in a preparation appropriately tailored to that route. Thus, the compounds described herein can be administered by injection, for example, intravenously, intramuscularly, intracutaneously, subcutaneously, or intraperitoneally. Accordingly, the compositions described herein may be included in pharmaceutical compositions and/or may form part of a pharmaceutically acceptable carrier or excipient.

Parenteral routes of administration include intravascular (intravenous, intraarterial), intramuscular, intraparenchymal, intradermal, subdermal, subcutaneous, intratumor, intraperitoneal, intrathecal, subdural, epidural, and intralymphatic injections that use a syringe and a needle or catheter.

The described compositions are injected in pharmaceutically acceptable carrier solutions. Pharmaceutically acceptable refers to those properties and/or substances which are acceptable to the mammal from a pharmacological/toxicological point of view. The phrase pharmaceutically acceptable refers to molecular entities, compositions, and properties that are physiologically tolerable and do not typically produce an allergic or other untoward or toxic reaction when administered to a mammal. The term pharmaceutically acceptable means able to be approved by a regulatory agency of the Federal or a state government or listed in the U.S. Pharmacopeia or other generally recognized pharmacopeia for use in animals and more particularly in humans.

As used herein, a "pharmaceutical composition" comprises a pharmacologically effective amount of an Active Pharmaceutical Ingredient (API, also therapeutic product, e.g., RNAi trigger), a pharmaceutically acceptable carrier, and optionally one or more a pharmaceutically acceptable excipients. Pharmaceutically acceptable excipients (excipients) are substances other than the API that have been appropriately evaluated for safety and are intentionally included in the drug delivery system. Excipients do not exert or are not intended to exert a therapeutic effect at the intended dosage. Excipients may act to a) aid in processing of the drug delivery system during manufacture, b) protect, support or enhance stability, bioavailability or patient acceptability of the API, c) assist in product identification, and/or d) enhance any other attribute of the overall safety, effectiveness, of delivery of the API during storage or use.

Excipients include, but are not limited to: absorption enhancers, anti-adherents, anti-foaming agents, anti-oxidants, binders, binders, buffering agents, carriers, coating agents, colors, delivery enhancers, dextran, dextrose, diluents, disintegrants, emulsifiers, extenders, fillers, flavors, glidants, humectants, lubricants, oils, polymers, preservatives, saline, salts, solvents, sugars, suspending agents, sustained release matrices, sweeteners, thickening agents, tonicity agents, vehicles, water-repelling agents, and wetting agents. A pharmaceutically acceptable excipient may or may not be an inert substance.

The pharmaceutical compositions described herein can contain other additional components commonly found in pharmaceutical compositions. The pharmaceutically-active materials may include, but are not limited to: anti-pruritics, astringents, local anesthetics, or antiinflammatory agents (e.g., antihistamine, diphenhydramine, etc.). It is also envisaged that cells, tissues or isolated organs that express or comprise the herein defined RNAi triggers may be used as "pharmaceutical compositions". As used herein, "pharmacologically effective amount," "therapeutically effective amount," or simply "effective amount" refers to that amount of an RNAi trigger to produce the intended pharmacological, therapeutic or preventive result.

Medicaments containing a tetrapeptide linker are also an object of the present invention, as are processes for the manufacture of such medicaments, which processes comprise bringing one or more compounds containing a tetrapeptide linker, and, if desired, one or more other therapeutically valuable substances into a galenical administration form.

### EXAMPLES

*Example 1. Synthesis of tetrapeptide linkers and conjugates having tetrapeptide linkers.*
A) Synthesis of peptide and AENA labeled peptides. 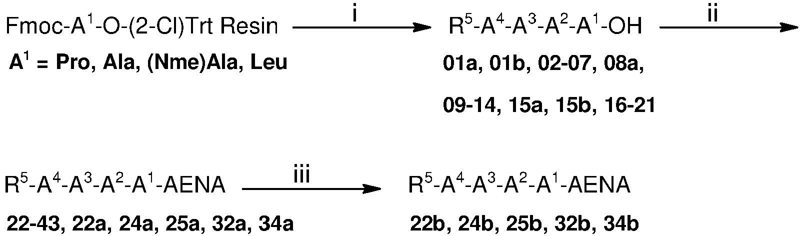
   *(i) Solid phase synthesis of compounds 1-21:* Peptide acids were synthesized from commercially available 2-Cl-Trt resin (EMD Millipore, Billerica, MA) pre-loaded with proline, leucine, alanine or N-methyl Alanine. Stepwise addition was performed using standard methods in the art of solid phase synthesis. Coupling was performed using PYBOP (4eq), amino acid (4eq), and DIEA (8eq). Fmoc deprotection was performed with 20% piperdine in DMF.
      After peptide synthesis the N-terminal amino acid was deprotected and coupled with the indicated R⁵ group. Coupling was performed using 2 eq of NHS activated esters of either NAG(OAc)₃ (as prepared in US 8802773 and Rozema et al. 2015) or PEG₁₂ (Quanta Biodesign, Plain City, OH, CAS #: 756525-94-7) in DMF containing 4eq of DIEA. Other R⁵ groups can be attached using similar syntheses. Following attachment of R⁵ the peptides were cleaved from resin using HFIP (30%) in DCM for 0.25 hours. After solvent removal the residue was triturated with Et₂O. Substrates were subsequently used without further purification.
   *(ii) General preparation of compounds 22-43 (22a, 24a, 25a, 32a, 34a):* To a solution of crude peptide substrate (**1-21**) was added PyBOP (2eq), DIEA (2ea), then AENA (2 eq) and stirred for 1 h at room temp. Upon completion solvent was removed in vacuo. Compounds **24a, 25a, 32a,** and **34a** were subsequently used without further purification. All other substrates were purified using HPLC with a Thermo Scientific Aquasil C18 reverse-phase column (250 x 21.2, Waltham, MA) eluting a gradient of acetonitrile and water buffered with 0.1% formic acid. After purification all compounds were dried by lyophilization.
   *(iii) Preparation of compounds **22b, 24b, 25b, 32b** and **34b**:* Compounds **24a** and **25a** were treated with 2% Hydrazine in DMF for 15 min and all solvents were subsequently removed in vacuo. Compound **32a** was treated with 50% TFA in DCM for 1 h and all solvents were subsequently removed in vacuo. Compound **34a** was treated with neat formic acid for 18 h and all solvents were subsequently removed in vacuo. A portion of unpurified compound **22a** was treated with MeOH (25%), H₂O (45%), and TEA (35%), stirred at room temp. overnight, and all solvents were subsequently removed in vacuo. All compounds, de-protected as described, were subsequently purified by HPLC as described in step (**ii**).
B. *General preparation of compounds 44a, 44b, 45a, 45b and 34b, Ester activation of peptide substrates (iv):* 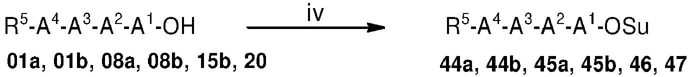 Crude compounds **01a, 01b, 08a, 08b, 15b,** and **20** were first purified on HPLC as described in step (ii). Prior to purification by HPLC compounds **01a** and **08a** were treated with MeOH (25%), H₂O (45%), and TEA (35%) as in the preparation of **22b** described in step (iii). Subsequently all compounds were activated as follows. In a flame dried flask, to a solution at 0°C, containing the purified substrate in DMF or DCM at 0.2 M concentration was added NHS (3eq) and DCC (3eq) and allowed to stir at room temperature under argon overnight. Upon completion the mixture was partially concentrated, chilled to -20°C, filtered and then all solvents were removed in vacuo. The residue was dissolved in a minimum of DCM and MeOH, precipitated into cold Et₂O and collected by decantation of the solvent after centrifugation. Precipitation into Et₂O was repeated until no residual dicyclohexylurea was detectable as confirmed by NMR. All prepared compounds were subsequently used without further purification.
C) *Synthesis of EDANS labeled substrates, General preparation of compounds 48-59:*

   H₂N-(CH₂)₂-O-(2-Cl)Trt Resin → H₂N-A²-A¹-PEG₄-Glu(EDANS)-NH(CH₂)₂-OH ***48-49***

   Each peptide acid was synthesized from commercially available 2-Cl-Trt resin (EMD Millipore, Billerica, MA) pre-loaded with ethanol amine. Stepwise solid phase synthesis was performed as described in step (i). After peptide synthesis the N-terminal FMOC was removed and the sequence cleaved from resin using 5% TFA, 5% H₂O in DCM for 0.5 hours and purified by HPLC as described in step (ii). Prior to purification compounds 52 and 53 were treated with 50% TFA, 5% TIS, 5% H₂O in DCM for 1 hour.
D) *Preparation of compound 60 (vi), Synthesis of control for digestion assay:* 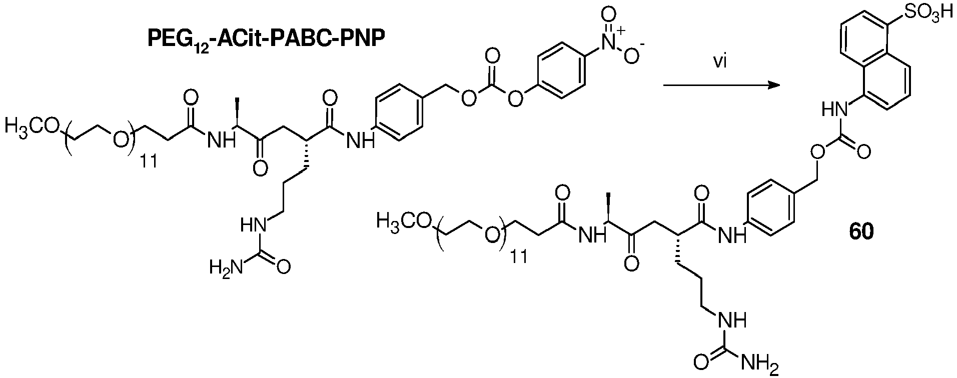
   To a solution containing PEG₁₂-ACit-PABC-PNP (0.052 mmol, 56 mg), prepared as previously described (US 8802733, Rozema et al. 2015, Carlson et al. 2015), and TEA (0.078 mmol, 10.8 µL) in DMF (400 uL) was added EDANS (0.052 mmol, 14 mg) and let to stir as a suspension at room temperature under argon overnight. Upon completion all solvents were removed in vacuo, the crude residue was triturated with Et₂O and purified using SiO₂ eluting a gradient of MeOH (10-20%) in CHCl₃. Yield 58 mg (92%).

**Table 1. Peptide Linkers.**

| Compound | R⁵ | A⁴ | A³ | A² | A¹ | *R⁶ |
|---|---|---|---|---|---|---|
| **01a** | NAG(OAc)₃ | Phe | Cit | Phe | Pro | OH |
| **01b** | PEG₁₂ | Phe | Cit | Phe | Pro | OH |
| **02** | PEG₁₂ | Phe | Lys(dde) | Phe | Pro | OH |
| **03** | PEG₁₂ | Phe | Lys(dde) | Phe | Leu | OH |
| **04** | NAG(OAc)₃ | Phe | Cit | Val | Pro | OH |
| **05** | PEG₁₂ | Phe | Cit | Phe | Leu | OH |
| **06** | NAG(OAc)₃ | Val | Cit | Phe | Pro | OH |
| **07** | PEG₁₂ | Phe | Cit | Phe | (Nme)Ala | OH |
| **08a** | NAG(OAc)₃ | Phe | Cit | Ala | Pro | OH |
| **08b** | PEG₁₂ | Phe | Cit | Ala | Pro | OH |
| **09** | PEG₁₂ | Ala | Cit | Phe | Pro | OH |
| **10** | PEG₁₂ | Phe | Asn(dmcp) | Phe | Pro | OH |
| **11** | NAG(OAc)₃ | Phe | Cit | Ala | Ala | OH |
| **12** | PEG₁₂ | Phe | Asp(O^{t}Bu) | Phe | Pro | OH |
| **13** | NAG(OAc)₃ | Ala | Cit | Ala | Ala | OH |
| **14** | NAG(OAc)₃ | Cit | Ala | Ala | Ala | OH |
| **15a** | NAG(OAc)₃ | Ala | Cit | Ala | Pro | OH |
| **15b** | PEG₁₂ | Ala | Cit | Ala | Pro | OH |
| **16** | PEG₁₂ | Gly | Phe | Leu | Gly | OH |
| **17** | NAG(OAc)₃ | Phe | Cit | Phe | Ala | OH |
| **18** | NAG(OAc)₃ | - | Cit | Ala | Ala | OH |
| **19** | NAG(OAc)₃ | - | Ala | Ala | Ala | OH |
| **20** | PEG₁₂ | - | Cit | Ala | Pro | OH |
| **21** | NAG(OAc)₃ | - | Ala | Cit | Ala | OH |
| **22a** | NAG(OAc)₃ | Phe | Cit | Phe | Pro | AENA |
| **22b** | NAG(OH)₃ | Phe | Cit | Phe | Pro | AENA |
| **23** | PEG₁₂ | Phe | Cit | Phe | Pro | AENA |
| **24a** | PEG₁₂ | Phe | Lys(dde) | Phe | Pro | AENA |
| **24b** | PEG₁₂ | Phe | Lys(NH₂) | Phe | Pro | AENA |
| **25a** | PEG₁₂ | Phe | Lys(dde) | Phe | Leu | AENA |
| **25b** | PEG₁₂ | Phe | Lys(NH₂) | Phe | Leu | AENA |
| **26** | NAG(OAc)₃ | Phe | Cit | Val | Pro | AENA |
| **27** | PEG₁₂ | Phe | Cit | Phe | Leu | AENA |
| **28** | NAG(OAc)₃ | Val | Cit | Phe | Pro | AENA |
| **29** | PEG₁₂ | Phe | Cit | Phe | (Nme)Ala | AENA |
| **30** | NAG(OAc)₃ | Phe | Cit | Ala | Pro | AENA |
| **31** | PEG₁₂ | Ala | Cit | Phe | Pro | AENA |
| **32a** | PEG₁₂ | Phe | Asn(dmcp) | Phe | Pro | AENA |
| **32b** | PEG₁₂ | Phe | Asn(NH₂) | Phe | Pro | AENA |
| **33** | NAG(OAc)₃ | Phe | Cit | Ala | Ala | AENA |
| **34a** | PEG₁₂ | Phe | Asp(O^{t}Bu) | Phe | Pro | AENA |
| **34b** | PEG₁₂ | Phe | Asp(OH) | Phe | Pro | AENA |
| **35** | NAG(OAc)₃ | Ala | Cit | Ala | Ala | AENA |
| **36** | NAG(OAc)₃ | Cit | Ala | Ala | Ala | AENA |
| **37** | NAG(OAc)₃ | Ala | Cit | Ala | Pro | AENA |
| **38** | PEG₁₂ | Gly | Phe | Leu | Gly | AENA |
| **39** | NAG(OAc)₃ | Phe | Cit | Phe | Ala | AENA |
| **40** | NAG(OAc)₃ | - | Cit | Ala | Ala | AENA |
| **41** | NAG(OAc)₃ | - | Ala | Ala | Ala | AENA |
| **42** | PEG₁₂ | - | Cit | Ala | Pro | AENA |
| **43** | NAG(OAc)₃ | - | Ala | Cit | Ala | AENA |
| **44a** | NAG(OH)₃ | Phe | Cit | Phe | Pro | OSu |
| **44b** | PEG₁₂ | Phe | Cit | Phe | Pro | OSu |
| **45a** | NAG(OH)₃ | Phe | Cit | Ala | Pro | OSu |
| **45b** | PEG₁₂ | Phe | Cit | Ala | Pro | OSu |
| **46** | PEG₁₂ | Ala | Cit | Ala | Pro | OSu |
| **47** | PEG₁₂ | - | Cit | Ala | Pro | OSu |
| **48** | H₂N | - | - | Phe | Pro | PGA |
| **49** | H₂N | - | - | Leu | Pro | PGA |
| **50** | H₂N | - | - | Val | Pro | PGA |
| **51** | H₂N | - | - | Phe | (Nme)Ala | PGA |
| **52** | H₂N | - | - | Asn(NH₂) | Pro | PGA |
| **53** | H₂N | - | - | Ser(OH) | Pro | PGA |
| **54** | H₂N | - | - | Phe | Leu | PGA |
| **55** | H₂N | - | - | Ala | Pro | PGA |
| **56** | H₂N | - | - | Phe | Ala | PGA |
| **57** | H₂N | - | - | - | Leu | PGA |
| **58** | H₂N | - | - | Gly | Pro | PGA |
| **59** | H₂N | - | - | - | Ala | PGA |
| **60** | PEG₁₂ | - | Ala | Cit | PABC | EDANS |

| | | | | | | |
|---|---|---|---|---|---|---|
| * where R6 = -OH, A¹ terminates in a carboxyl group (i.e., -OH replaces -NH-) | | | | | | |

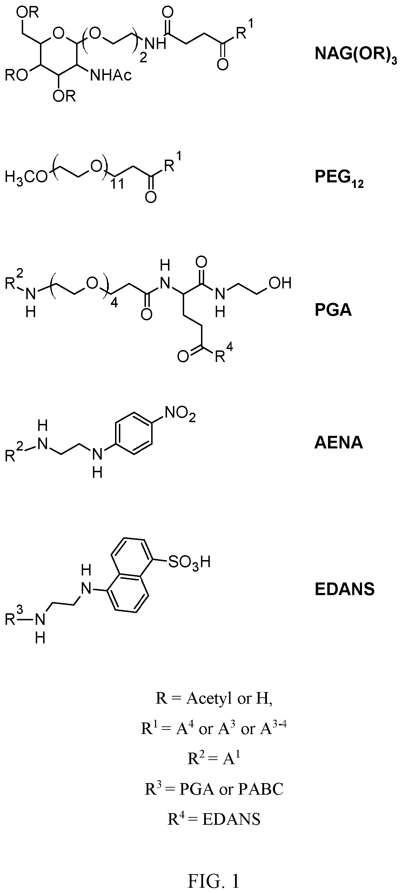

Structures for some components are shown in FIG. 1.

*Example 2. Rate of cleavage of tetrapeptide linkers.*

Various amino acid combinations, tetrapeptides, were used to link various first moieties (R⁵) and an AENA report group second compound (R⁶) to form assay substrates (substrates). Cleavage rate of the peptides were evaluated by analysis of digestion of the substrates in the presence of rat lysosomal extract.

Reporting groups were coupled to the C-terminus of the peptide substrates as described above for UV-vis monitoring to explore the influence of amino acid sequence on kinetics of peptide degradation by HPLC. N-(2-aminoethyl)-4-nitroaniline (AENA) or N-(aminoethyl)-5-napthylamine-1-sulfonic acid (EDANS) were used as reporting groups for HPLC monitoring as neither absorptivity nor lambda maximum were affected for either after cleavage from the peptide. The N-terminus of each peptide substrate was modified with either methoxy polyethylene glycol (PEG₁₂) or N-acetyl galactosamine (NAG(OR)₃) (FIG. 1).

*Digestion Assay:* Proteolytic digestion experiments were carried out at a fixed substrate concentration (0.51 µmol) using liver lysosomal (enzyme) extract (0.45 µg/µL) in a 25mM MES solution buffered to pH 5 containing 1% CHAPS and 1.8mM DTT incubated at 37°C. Enzyme extract was activated in the presence of DTT for 15 minutes at 37°C prior to addition of AENA labeled peptide substrates. At various time points, up to 26 h, 20 µL aliquots were removed and acidified to a pH of 3 with TFA. Analysis of fractions was performed using HPLC with a Aquasil C18 reverse-phase column (250 x 4.6, Thermofisher, Waltham, MA), monitored at 390 nm and 335 nm for AENA and EDANS respectively. Elution was performed with a gradient of acetonitrile and water buffered with 0.1% formic acid. Generation of unmodified reporter molecule was measured. To minimize freeze thaw cycles the lysosomal extract was dispensed into multiple aliquots prior to storage at -80°C. A new aliquot was employed for each study and used immediately upon thawing. Lysosomal extract was sourced from female Hans Wistar rats by differential density gradient centrifugation (Graham et al. 2000). Protein concentration of lysosomal extract was determined by standard BCA protein assay protocols. Compound **60** was used for normalization of velocity between experiments (Tables 2-4). The average experimental velocity observed for **60** over multiple assays was 123.8 nmol/h. Normalized sample velocity was calculated as: [(average compound **60** velocity ÷ experimental compound **60** velocity)] × sample experimental velocity.

Rate (also called velocity of degradation or velocity) was extrapolated during early linear turnover (Table 2). As used herein, rate is defined as the linear rate of reporting group generation at less than 50% completion (digestion rate was assumed to be linear at less than 50% completion). For substrates in which cleavage deviated from consecutive first order kinetics, the percent of reporting group liberated at the endpoint of digestion was used as a metric for comparison.

The date in Table 2 indicate that fastest rates of cleavage were observed with tetrapeptides having bulky hydrophobic amino acids and positions A² and A⁴, bulky hydrophilic (polar) amino acids at position A³, and proline or leucine at position A¹. In some embodiments, the amino acid at position A¹ is proline.

Tetrapeptide linkers having proline at the A¹ position had the highest velocities. If the proline of compounds **30** (FCitAP (SEQ ID NO: 8)), and **23** (FCitFP (SEQ ID NO: 12)) was changed to alanine, compounds **33** (FCitAA (SEQ ID NO: 7)) and **39** (FCitFA (SEQ ID NO: 10)), a dramatic reduction in rate and time to completion of digestion was observed. Substituting leucine at A¹ in compound **27** with proline, as shown in compound **23,** resulted in a considerable increase in velocity for the proline-containing tretrapeptide. A small increase in velocity was also observed the proline-containing compound **24b** compared to the when leucine-containing compound **25b.**

Tetrapeptides having larger (bulky) hydrophobic amino acids at positions A² and A⁴ also generally exhibited higher velocities. Increasing side chain bulk at the A² position, compounds **30, 26** and **23,** increased velocity of digestion. Similarly, increasing side chain bulk at the A⁴ position increased velocity of digestion, as shown in compounds **31, 28** and **23,** respectively.

Tetrapeptides having bulky polar (neutral or positively charged) amino acids at position A³ exhibited faster rates of cleavage, see compounds **23, 24b, 32b,** and **34b.**

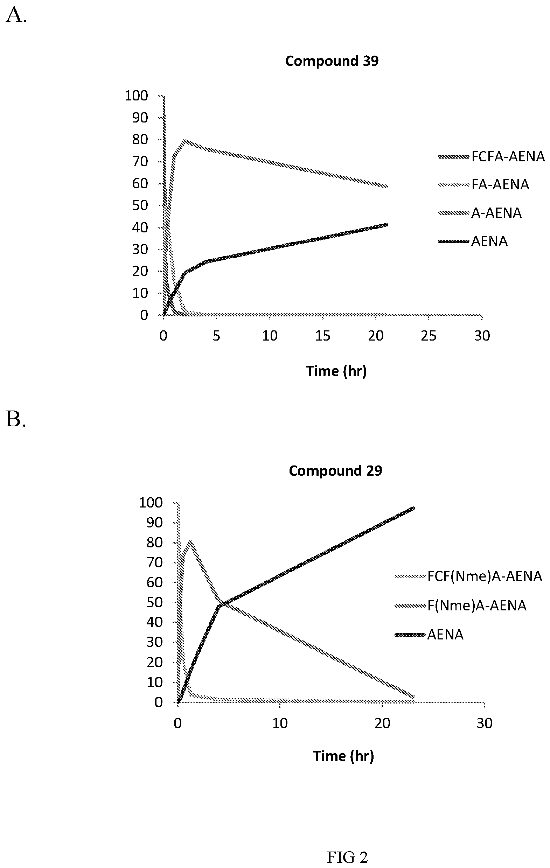

N-methylation of compound **39** at A¹, to yield compound **29,** resulted in a faster rate and faster completion of digestion (see also FIG. 2).

**Table 2. Cleavage velocity of various peptide linkers. Various peptides were synthesized and cleavage velocity was determined.**

| Assay Substrate | Peptide Sequence | SEQ ID NO: | **(60)** Velocity nmol/hr | Sample Velocity nmol/hr | Normalized Velocity nmol/hr | % Released at Endpoint (21 hr) |
|---|---|---|---|---|---|---|
| **(23)** PEG₁₂-**FCitFP**-AENA | FCitFP | 12 | 123.6 | 192.7 | 192.9 | *99.5 |
| **(24b)** PEG₁₂-**FKFP**-AENA | FKFP | 16 | 134.7 | 134.8 | 123.9 | ***98.9 |
| **(25b)** PEG₁₂-**FKFL**-AENA | FKFL | 15 | 123.5 | 117.0 | 117.4 | **99.8 |
| **(26)** NAG(OAc)₃-**FCitVP**-AENA | FCitVP | 13 | 144.3 | 119.5 | 102.5 | 97.8 |
| **(27)** PEG₁₂-**FCitFL**-AENA | FCitFL | 11 | 123.5 | 94.5 | 94.7 | **99.7 |
| **(28)** NAG(OAc)₃-**VCitFP**-AENA | VCitFP | 19 | 137.8 | 84.3 | 75.8 | *99.4 |
| **(29)** PEG₁₂-**FCitF(Nme)A-**AENA | FCitF(Nme)A | 9 | 123.6 | 62.5 | 62.6 | *97.3 |
| **(30)** NAG(OAc)₃-**FCitAP**-AENA | FCitAP | 8 | 108.4 | 54.3 | 62.0 | 96.5 |
| **(31)** PEG₁₂-**ACitFP-**AENA | ACitFP | 3 | 123.6 | 49.5 | 49.6 | *98.9 |
| **(32b)** PEG₁₂-**FNFP**-AENA | FNFP | 17 | 134.7 | 42.0 | 38.6 | ***99.3 |
| **(33)** NAG(OH)₃-**FCitAA**-AENA | FCitAA | 7 | 137.8 | - | - | 68.5 |
| **(34b)** PEG₁₂-**FDFP**-AENA | FDFP | 14 | 134.7 | 18.0 | 16.6 | ***66.5 |
| **(35)** NAG(OAc)₃-**ACitAA**-AENA | ACitAA | 1 | 120.0 | - | - | 49.8 |
| **(36)** NAG(OAc)₃-**CitAAA-**AENA | CitAAA | 5 | 100.9 | - | - | **47.7 |
| **(37)** NAG(OAc)₃-**ACitAP**-AENA | ACitAP | 2 | 108.0 | 11.8 | 13.5 | 44.2 |
| **(38)** PEG₁₂-**GFLG**-AENA | GFLG | 18 | 127.4 | 10.0 | 9.7 | **47.8 |
| **(39)** NAG(OAc)₃-**FCitFA**-AENA | FCitFA | 10 | 108.4 | - | - | 40.5 |
| **(40)** NAG(OAc)₃-**CitAA-**AENA | CitAA | | 120.0 | 3.7 | 3.9 | 15.6 |
| **(41)** NAG(OAc)₃-**AAA**-AENA | AAA | | 120.0 | 1.6 | 1.6 | 8.2 |
| **(42)** PEG₁₂-**CitAP**-AENA | CitAP | | 135.2 | 1.0 | 0.9 | **5.8 |
| **(43)** NAG(OAc)₃-**ACA**-AENA | ACitA | | 120.0 | - | - | 1.1 |

| | | | | | | |
|---|---|---|---|---|---|---|
| * Endpoint at 23 hours ** Endpoint at 24 hours *** Endpoint at 26 hours. (compound numbers and amino acids are in bold) | | | | | | |

For a given tetrapeptide, rate of cleavage (rate of liberation of unmodified H²N-R⁶) was not significantly affected by the identity of compound R⁵. Compounds **22a** and **23** were essentially equivalent in terms of rate of proteolytic digestion (Table 3). Similarly it was shown that acetyl modified NAG(OAc)₃, compound **22a,** had a negligible effect on the velocity of digestion when compared to the unmodified analog, compound **22b** (Table 3).

**Table 3. Cleavage velocity of FCitFP (SEQ ID NO: 12) linker having various N-terminal (R⁵) (compounds 23, 22a, and 22b).**

| Assay substrate | Velocity nmol/h | | |
|---|---|---|---|
| | compound **60** | Sample | Normalized |
| **(23)** PEG₁₂-**FCitFP**-AENA | 123.6 | 192.7 | 192.9 |
| **(22a)** NAG(OAc)₃-**FCitFP**-AENA | 144.0 | 244.9 | 210.5 |
| **(22b)** NAG(OH)₃-**FCitFP**-AENA | 137.8 | 223.7 | 201.0 |

*Example 3. Effect of amino acids A¹ and A² on rate of digestion of dipeptides.* Dipeptide substrates having amine termini and a C-terminal (R⁶) EDANS reporters, were made to further analyze the effects of amino acids A¹ and A² on cleavage rate. The dipeptides substrates mimic the tetrapeptide linkers following a first cleavage event of the tetrapeptide between amino acids A² and A³. The dipeptides were treated with extracts and digestion products were analyzed as described above. As seen in Table 4, dipeptides having a proline at A¹ exhibited the fasted cleavage rate. In agreement with the data in Table 2, cleavage rate was found to be dependent on the amino acid at position A¹ and the size and charge of the amino acid as position A² (Table 4). Peptides with proline at position A¹ has the greatest rate of cleavage. Rate of cleavage also increased with increasing size and hydrophobicity of the A² amino acid, as seen for compounds **48, 49, 50, 52, 55** and **58.**

Addition of phenylalanine to compounds **57** and **59** (to yield compounds **54** and **56,** respectively) resulted in a substantial increase in the velocity of digestion despite the additional amino acid.

**Table 4. Cleavage velocity of dipeptide EDANS C-terminal modified substrates.**

| Compound Name | Substrate Sequence | (60) Velocity nmol/hr | Sample Velocity nmol/hr | Normalized Velocity nmol/hr |
|---|---|---|---|---|
| **(48)** H₂N-FP-PEG₄-Glu(EDANS)-NH-(CH₂)₂-OH | F-P | 145.0 | 448.5 | 383.0 |
| **(49)** H₂N-LP-PEG₄-Glu(EDANS)-NH-(CH₂)₂-OH | L-P | 127.6 | 349.1 | 338.7 |
| **(50)** H₂N-VP-PEG₄-Glu(EDANS)-NH-(CH₂)₂-OH | V-P | 135.2 | 213.5 | 195.5 |
| **(51)** H₂N-F(Nme)A-Glu(EDANS)-NH-(CH₂)₂-OH | F-(Nme)A | 135.2 | 177.6 | 162.6 |
| **(52)** H₂N-NP-PEG₄-Glu(EDANS)-NH-(CH₂)₂-OH | N-P | 127.6 | 156.5 | 151.9 |
| **(53)** H₂N-SP-PEG₄-Glu(EDANS)-NH-(CH₂)₂-OH | S-P | 127.6 | 145.0 | 140.7 |
| **(54)** H₂N-FL-PEG₄-Glu(EDANS)-NH-(CH₂)₂-OH | F-L | 145.0 | 155.0 | 132.4 |
| **(55)** H₂N-AP-PEG₄-Glu(EDANS)-NH-(CH₂)₂-OH | A-P | 135.2 | 125.7 | 115.1 |
| **(56)** H₂N-FA-PEG₄-Glu(EDANS)-NH-(CH₂)₂-OH | F-A | 135.2 | 61.3 | 56.1 |
| **(57)** H₂N-L-PEG₄-Glu(EDANS)-NH-(CH₂)₂-OH | L | 135.2 | 58.3 | 53.3 |
| **(58)** H₂N-GP-PEG₄-Glu(EDANS)-NH-(CH₂)₂-OH | G-P | 127.6 | 14.4 | 13.9 |
| **(59)** H₂N-A-PEG₄-Glu(EDANS)-NH-(CH₂)₂-OH | A | 135.2 | 7.9 | 7.2 |

*Example 4. Analysis of digestion using extracts from different sources.* The observed rates of cleavage were not dependent on the source of proteolytic enzymes. The order of increasing velocity (**23>30>37>42**) was maintained when digested using commercially available rat and human liver S9 extract (Xenotech LLC, Lenexa, KS) (Table 5). Thus rates of cleavage are expected to be similar across different species and cell types.

**Table 5. Relative velocity of substrates in different extracts.**

| Compound Name | Substrate Sequence (SEQ ID NO:) | Relative* Velocity | | |
|---|---|---|---|---|
| | | Lysosomal Extract | RAT S9 Extract | HUMAN S9 Extract |
| **(23)** PEG₁₂-**FCFP**-AENA | FCitFP (12) | 100.0 | 100.0 | 100.0 |
| **(30)** NAG(OAc)₃-**FCitAP**-AENA | FCitAP (8) | 32.1 | 28.3 | 45.7 |
| **(37)** NAG(OAc)₃-**ACitAP**-AENA | ACitAP (2) | 7.0 | 3.3 | 7.1 |
| **(42)** PEG₁₂-**CitAP**-AENA | CitAP | 0.5 | 0.6 | 1.7 |

| | | | | |
|---|---|---|---|---|
| *Normalized to compound **23.** | | | | |

*Example 5. Analysis of peptide linker digestion intermediates.* Aliquots from examples 2 and 3 were further analyzed by HPLC and electro-spray ionization mass spectrometry to identify digestion intermediates. The analysis indicated multiple scissile locations for the initial endoproteolytic event of substrate degradation. Tetrapeptide linkers having a proline at position A¹ (**22a, 22b, 23, 24b, 26, 28, 30, 31, 34b, 37,** and **42**) did not appear to generate a significant amount of H₂N-Pro-R⁶ intermediate, i.e. there did not appear to be significant digestion between proline at A¹ and the amino acid at position A². Only A²-Proline-AENA or free AENA was observed. When A¹ was alanine or leucine, the rate limiting step was proteolysis of the final residue. Digestion intermediates are shown for Compounds **39** (FCitFA (SEQ ID NO: 10)) and **29** (FCitF(Nme)A (SEQ ID NO: 9)) in FIG. 2.

*Example 6. Release of H₂N-R⁶ was not impaired by steric bulk of a polymeric compound at R⁶.*

PEG tetrapeptide modifying agents were synthesized by using a PEG group at R⁵ and an activated ester at R⁷ (formula V). Membrane active polyamines (see below) were modified with Factor VII siRNA, CDM-NAG and the indicated tetrapeptide (**45b, 46**) or tripeptide control (**47**) modifying agents as previously described (US Patents 8137695 and 8426554) to form a dynamic polyconjugate delivery polymer. 1 mg/kg polymer conjugated to 0.25 mg/kg siRNA was then injected into mice. At day 5, samples were collected and assayed for Factor VII. Knockdown of Factor VII requires cleavage of the modifying agent from the polyamine at a rate similar to the previously described CDM masking agents and dipeptide-PABC masking agents. As shown in Table 6, the described tetrapeptide agents (**45b, 46**) were effective modifying agents when used with DPC-mediated siRNA delivery (US Patents 8137695 and 8426554), indicating rapid cleavage in the cell to release the membrane active polyamine. Effective in vitro knockdown also indicates that the presence of the PEG group did not adversely affect digestion of the linker in vivo. For dynamic polyconjugate delivery polymers modified with PEG₁₂ functionalized tetrapeptides, the order of increasing knockdown in vivo (**45b>46>47**) correlated with in vitro kinetic results. Validation of the in vitro model for kinetic evaluation was shown by the results obtained in animal studies.

**Table 6. Normalized Factor VII levels after injection of polymer-siRNA conjugates targeted with acid-labile CDM-NAG¹¹.**

| PEG-peptide modifying agent | Relative F VII activity |
|---|---|
| **(45b)** PEG-FCitAP-OSu | 40±06 |
| **(46)** PEG-ACitAP-OSu | 66±28 |
| **(47)** PEG-CitAP-OSu | 97±34 |

*Example 7. In vivo analysis of lability.* The tetrapeptides were analyzed for in vivo lability and reversibility by testing the linkers as modifying agents for DPC-mediated siRNA delivery.

### A) RNA interference triggers (siRNA).

*siRNA synthesis:* The control siRNAs of the following sequences were synthesized using standard phosphoramidite chemistry.
siF7 sense: 5'-(NH₂-C6)-GfcAfaAfgGfcGfuGfcCfaAfcUfcAf(invdT)-3' SEQ ID NO: 4
siF7 antisense: 5'dTsGfaGfuUfgGfcAfcGfcCfuUfuGfcdTsdT-3' SEQ ID NO: 6
(lower case, 2'-OMe substitution; f, 2'-F substitution; d, 2'-deoxy substitution; and s, phosphorothioate linkage.)

*Modifications of amine siRNA with DBCO disulfide:* DBCO-modified siRNAs were synthesized by reaction of 5' amine-modified siRNA with 1 weight equivalents (wt. eq.) of dibenzocyclooctyne-S-S-N-hydroxysuccinimidyl ester (ALDRICH catalogue #761532) and 0.36 wt. eq. of NaHCO₃ in water at 4°C for 16 hours. The modified siRNAs were then precipitated by the addition of 9 volumes of ethanol and incubation at -80°C for 2 h. The precipitate was dissolved in RNase-free PBS buffer and quantified by measuring absorbance at the 260 nm.

### B) Polymer synthesis:

*RAFT copolymer of N-Boc-ethoxyethylamine acrylate and sec-butyl acrylate (EAB):* Solutions of AIBN (1.00 mg/mL) and RAFT agent CPCPA (10.0 mg/mL) in butyl acetate were prepared. Monomer molar feed was 55% N-Boc-ethoxyethylamine acrylate, 45% sec-butyl acrylate (CAS # 2998-08-5). Theoretical Mw was 100,000.

N-Boc-ethoxyethylamine acrylate (0.890 g, 3.43 mmol) sec-butyl acrylate (0.391 mL, 0.360 g, 2.81 mmol) CPCPA solution (0.350 mL, 0.0125 mmol), AIBN solution (0.308 mL, 0.00188 mmol), and butyl acetate (5.3 mL) were added to a 20 mL glass vial with stir bar. The vial was sealed with a septa cap and the solution bubbled with nitrogen using a long syringe with a second syringe as the outlet for 1 h. The syringes were removed and the vial heated to 80°C for 16 h using an oil bath. The solution was allowed to cool to room temperature and transferred to a 50 mL centrifuge tube before hexane (35 mL) was added to the solution. The solution was centrifuged for 2 min at 4,400 rpm. The supernatant layer was carefully decanted and the bottom (solid or gel-like) layer was rinsed with hexane. The bottom layer was then re-dissolved in DCM (7 mL), precipitated in hexane (40 mL) and centrifuged once more. The supernatant was decanted and the bottom layer rinsed with hexane before the polymer was dried under reduced pressure for several hours. Yield of crude EAB copolymer was 0.856 g. Samples of the crude polymer were taken for multi-angle light scattering (MALS). The dried, crude copolymer was dissolved in DCM (100 mg/mL). Hexane was added until just after the cloud point was reached. The resulting milky solution was centrifuged. The bottom layer was extracted and fully precipitated into hexane. The fraction was centrifuged, after which the copolymer was isolated and dried under vacuum. Yield of isolated fraction of EAB copolymer was 0.478 g. Samples of the fractionated copolymer were taken for ¹H-NMR and MALS. Composition determined by ¹H-NMR was 61% N-Boc-ethoxyethylamine and acrylate, 39% sec-butyl acrylate.

*MALS Analysis:* Approximately 10 mg of the copolymer was dissolved in 0.5 mL 75% dichloromethane, 20% tetrahydrofuran, 5% acetonitrile. The molecular weight and polydispersity (PDI) were measured using a Wyatt Heleos II multiangle light scattering detector attached to a Shimadzu Prominence HPLC using a Jordi 5µ 7.8×300 Mixed Bed LS DVB column. Crude Polymer: MW: 59,000 (PDI 1.3), Fractionated Polymer: MW 70,000 (PDI: 1.1).

*Deprotection*/*Dialysis:* The dried samples were treated with 2M HCl in acetic acid (∼7 ml) for 1 h to remove the BOC protecting groups. Then the reaction was diluted with 20 ml of water and allowed to stir for 10-15 min. The fractions were then dialyzed with 3500 MW dialysis tubing in high salt, high salt, and water for 15h, 8h, and 15h respectively. The fractions were then transferred to 50 ml centrifuge tubes and lyophilized for 3 days or until dry. The dry samples were brought up at 20 mg/ml in water for further study.

*C) Formation of reversibly modified siRNA-polyamine conjugate:* Polyacrylate EAB in 5 mM pH 8.0 HEPES buffer was modified 1.5 wt% with a N-hydroxysuccinimidyl activated PEG₄ azide (Azido-dPEG₄-NHS ester from Quanta Biodesign) to provide azide groups for subsequent attachment of siRNA. The azide-modified polymer was then diluted to 5 mg/mL in 60 mg/mL HEPES base. To this solution was added 15 mg/mL (3 wt equivalents) of the indicated protease-cleavable PEG modifying reagents (the described herein tetrapeptide agents or PEG₁₂-FCit-PABC-PNP (8426554) to modify 40-50% of available amine groups. After 1 h, DBCO-modified rodent factor VII siRNA (0.25 wt eq relative to polymer) was added to polymer solution. After incubation overnight, conjugates were further modified by addition of molar excess relative to available amine groups of an N-acetylgalactosamine derivative CDM-NAG, NAG-A⁴-A³-A²-A¹-NHS or NAG-ACit-PABC-PNP (US Patent 8426554, Rozema et al. 2015, Carlson et al. 2015), and incubated for 30 min to reversibly modify remaining polymer EAB amines.

### D) In vivo assay.

*Mice and injection procedures.* Male Wistar Han rats and female ICR mice, 6 to 8 weeks old, were obtained from Harlan Sprague-Dawley, (Indianapolis, IN). Animals were handled in accordance with animal used protocols approved by the Animal Care and Use Committee at Arrowhead Madison Inc. Rodents were maintained on a 12 hours light/dark cycle with free access to water and food (Harlan Teklad Rodent Diet, Harlan, Madison, WI). Animals were injected with 1 mg/kg polymer conjugated to 0.25 mg/kg siRNA. Samples were collected 5 days post injection.

*Serum FVII activity measurements.* Serum samples were prepared by collecting blood by submandibular bleeding into microcentrifuge tubes containing 0.109 M sodium citrate anticoagulant (1 volume) following standard procedures. FVII activity in serum was measured with a chromogenic method using a test kit (BIOPHEN VII, Aniara, Mason, OH) following manufacturer's recommendations. Absorbance of colorimetric development was measured using a Tecan Safire- 2 microplate reader at 405 nm.

The data in Table 7 show the tetrapeptide modified DPC delivery polymers were effective in delivery of siRNA in vivo. Further, the tetrapeptide agents were as effective as the previously described dipeptide-PABC modifying agents. Conjugates bearing **44a** and **44b,** containing the proteolytic FCitFP (SEQ ID NO: 12) tetramer, displayed equivalent activity to conjugates utilizing PABC mediated degradation for knockdown (Table 7). Effective siRNA delivery indicates effective modification and targeting of the polyamine and successful digestion and release of the polymer in hepatocytes after delivery to the cell.

In vivo activity and function correlated with in vitro digestion velocity data with FCitFP (SEQ ID NO: 12) modified DPC delivery polymers, showing better siRNA delivery than FCitAP modified DPC delivery polymers. Thus, the in vitro velocity of digestion data accurately predicted in vivo digestion and release data.

**Table 7. Normalized Factor VII levels after injection of polymer-siRNA conjugates targeted with protease-labile NAG reagents.**

| modifying agent | Relative Factor VII activity |
|---|---|
| Activity in mice | |
| **(44a)** PEG-FCitFP-OSu/(**44b**) NAG-FCitFP-OSu | 27±06 |
| **(45a)** PEG-FCitAP-OSu/(**45b**) NAG-FCitAP-OSu | 48±14 |
| PEG-FCit-PABC-PNP/NAG-ACit-PABC-PNP | 24±11 |

| Activity in rats | |
|---|---|
| **(44a)** PEG-FCitFP-OSu/(**44b**) NAG-FCitFP-OSu | 26±22 |
| **(45a)** PEG-FCitAP-OSu/(**45b**) NAG-FCitAP-OSu | 48±8 |
| PEG-FCit-PABC-PNP/NAG-ACit-PABC-PNP | 30±13 |

*Example 8. RGD mimic tetrapeptides and PEG-tetrapeptide modified DPC delivery polymers.*
A) *Synthesis of APN 1170-100A (100A) and APN 1203-006 (006) amphipathic membrane active polyamines.*

| Polymer | MW (protected) | Theoretical MW (deprotected) | PDI | % Amine Incorp. | % Alkyl Incorp. | % End Group Removal | Azides/ Polymer |
|---|---|---|---|---|---|---|---|
| APN 1170-100A | 64,430 | 45,765 | 1.22 | 56 | 44 | 0 | 1.25 |
| APN 1203-006 | 60,330 | 43,578 | 1.05 | 56 | 44 | 99 | 1.14 |

i) *Materials.* 2,2'-Azobis(2,4-dimethyl valeronitrile) (V-65, radical initiator) was purchased from Wako Pure Chemical Industries. Propyl acrylate was purchased from Polysciences Inc. N-Boc-ethoxy-ethylamine acrylate was obtained from WuXi Inc. 2-(Dodecylthiocarbonothioylthio)-2-methylpropionic acid (DDMAT, RAFT Agent), 1,1'-Azobis-(cyclohexanecarbonitrile) (ACHN), 1-Ethylpiperidine hypophosphite (EPHP), Pentafluorophenol, N,N'-Dicyclohexylcarbodiimide and N,N-diisopropyl-ethylamine were purchased from Sigma Aldrich. O-(2-Aminoethyl)-O'-(2-azidoethyl)triethylene Glycol (azido-PEG₄-amine) was purchased from Biomatrik Inc.
ii). *RAFT copolymer of N-Boc-ethoxyethylamine acrylate and propyl acrylate (EAP).* Solutions of V-65 (2 mg/mL) and RAFT agent DDMAT (10 mg/mL) in butyl acetate were prepared. Monomer molar feed was 52% N-Boc-ethoxyethylamine acrylate, 48% propyl acrylate. Theoretical Mw was 75,000. RAFT agent (DDMAT) to initiator (V-65) molar ratio was 6.67:1.
   N-Boc-ethoxyethylamine acrylate (1.778 g, 6.86 mmol), propyl acrylate (0.794 mL, 0.722 g, 6.33 mmol), DDMAT solution (1.215 mL, 0.0333 mmol), V-65 solution (0.621 mL, 0.005 mmol), and butyl acetate (10.2 mL) were added to a 20 mL glass vial with a stir bar. The vial was sealed with a septa cap and the solution bubbled with nitrogen using a long needle with a second needle as the outlet for 1 h. The needles were removed and the vial was heated to 50°C for 24h with stirring. The solution was allowed to cool to room temperature and transferred equally between two 50 mL centrifuge tube before hexane (35 mL) was added to both tubes. The solution was centrifuged for 2 min at 4400 rpm. The supernatant layer was carefully decanted and the bottom layer rinsed with hexane. The bottom layer of each tube was then re-dissolved in dichloromethane (7 mL), precipitated in hexane (40 mL) and centrifuged once more. The supernatant was decanted and the bottom layer rinsed with hexane before the layers were combined to one 50 mL centrifuge tube and the polymer was dried under reduced pressure for several hours. The yield of crude EAP copolymer was 2.1 g. Samples of the copolymer were taken for multi-angle light scattering (MALS), and ¹H-NMR.
   Polymer 006: The composition determined by ¹H-NMR was 55% N-Boc-ethoxyethylamine acrylate and 45% propyl acrylate. The Mw for 006 determined by MALS was 58,600 g/mol with a polydispersity index (PDI) of 1.04.
   Polymer 100A: Composition by 1H-NMR: 56% N-Boc-ethoxyethylamine acrylate and 44% propyl acrylate. MW by MALS: 65,150, PDI of 1.122.
iii). *Radical induced ω-end group removal (polymer 006 only).* Solutions of 1,1'-Azobis-(cyclohexanecarbonitrile) (ACHN, 20 mg/mL) and 1-Ethylpiperidine hypophosphite (EPHP, 100 mg/mL) were prepared in toluene. EAP (2 g, 0.035 mmol), ACHN (0.213 mL, 0.5 eq, 0.0174 mmol), EPHP (1.25 mL, 20 eq, 0.697 mmol), and toluene (25.2 mL) were added to a 40 mL glass vial with a stir bar. The vial was sealed with a septa cap and the solution bubbled with nitrogen using a long needle with a second needle as the outlet for 1 h. The needles were removed and the vial was heated to 100°C for 2 h. The solution was allowed to cool to room temperature and -20 mL toluene was removed by rotary evaporation. The remaining solution was transferred to a 50 mL centrifuge vial, and hexane (35 mL) was added. The solution was centrifuged for 2 min at 4400 rpm. The supernatant layer was carefully decanted and the bottom layer rinsed with hexane. The bottom layer was then re-dissolved in dichloromethane (7 mL), precipitated in hexane (40 mL) and centrifuged once more. The supernatant was decanted and the bottom layer rinsed with hexane before the polymer was dried under reduced pressure for ∼ 1 h. The polymer was dissolved in methyl tert-butyl ether (80 mL) and transferred to a separatory funnel. The solution was then washed with 3 × 30 mL volumes of H₂O followed by 3 × 30 mL volumes of saturated NaCl. The polymer solution was then dried over sodium sulfate, and vacuum filtered through 0.45 µm GHP filters. MTBE was removed via rotary evaporation and high vacuum. A sample was taken for monitoring of end group removal using a UV spectrophotometer. End group removal was calculated to be 99%. Samples were taken for MALS, GC-FID, and ¹H-NMR. The composition of 006 by ¹H-NMR was 55% N-Boc-ethoxyethylamine acrylate and 45% propyl acrylate. The conversion of 006 determined by GC-FID was 81.4% for the N-Boc-ethoxyethylamine acrylate and 77.3% for the propyl acrylate. The conversion of 100A determined by GC-FID conversion was 87% for N-Boc-ethoxyethylamine acrylate and 83% for propyl acrylate. The Mw for polymer 006 determined by MALS was 57,700 g/mol with a polydispersity index (PDI) of 1.06.
iv). *Pentafluorophenol activation of α-end group.* EAP polymer (2 g, 0.0347 mmol), pentafluorophenol (63.8 mg, 0.3466 mmol), N,N'-Dicyclohexylcarbodiimide (71.5 mg, 0.3466 mmol), and dichloromethane (40mL) were added to a 100 mL round bottom flask with a stir bar. The flask was stoppered with a rubber septum and the system was purged with nitrogen for 15 min. The solution was stirred for 16 h at room temperature. Additional Pentafluorophenol (63.8 mg, 0.3466 mmol) and N,N'-Dicyclohexylcarbodiimide (71.5 mg, 0.3466 mmol) were added, the flask stoppered with a rubber septum, and the system was purged with nitrogen for 15 min. The solution was stirred for 3 h at room temperature. The polymer was precipitated with hexane (∼10× volume), centrifuged, and the solvent was decanted. The polymer was dissolved in minimal dichloromethane, precipitated with hexane (∼10× volume), centrifuged, and the solvent was decanted. The polymer was dissolved in minimal ethyl acetate, precipitated with hexane (∼10× volume), centrifuged, and the solvent was decanted. The polymer precipitate was dried under high vacuum until the solid reached a constant weight.
v). *Azide functionalization of α-end group.* In a 100 ml round bottom flask equipped with a rubber septum and stir bar, polymer from the previous step (1.9 g, 0.0329 mmol) was dissolved in dichloromethane (38 mL). Azido-PEG₄-Amine (86.4 mg, 0.3293 mmol) and N,N-Diisopropylethylamine (46.8 mg, 63.1 µL, 0.3622 mmol) were added to the flask with stirring. The system was purged with nitrogen for 15min, and the reaction was left to stir at room temperature overnight. Additional Azido PEG₄ Amine (86.4 mg, 0.3293 mmol) and N,N-Diisopropylethylamine (46.8 mg, 63.1 µL, 0.3622 mmol) were added to the flask, the system was purged with N₂ gas, and the reaction was stirred for 3h at room temperature. The polymer was precipitated with hexane (∼10× volume), centrifuged, and the solvent was decanted. The polymer was dissolved in minimal dichloromethane, precipitated with hexane (∼10× volume), centrifuged, and the solvent was decanted. The polymer precipitate was dried under high vacuum until the solid reached a constant weight. The yield of Azide functionalized EAP was 1.77g. Samples of the copolymer were taken for multi-angle light scattering (MALS), and ¹H-NMR.
   Polymer 006: The composition determined by ¹H-NMR was 56% N-Boc-ethoxyethylamine acrylate and 44% propyl acrylate. The Mw determined by MALS was 60,330 g/mol with a polydispersity index (PDI) of 1.05.
   Polymer 100A: The composition by ¹H-NMR was 56% N-Boc-ethoxyethylamine acrylate and 44% propyl acrylate. The Mw determined by MALS: 64,430 with PDI of 1.217.
   Mono-Azide: The term "mono-azide" or "mono-azide polymer" indicates that steps D and E of the procedures above were done and an azide group was coupled to the α-end group of the polymer.
vi). *Boc Deprotection and Tangential Flow Filtration.* In a 100 mL round bottom flask, 2M HCl in acetic acid (28mL) was added to Azide functionalized EAP copolymer (1.67g, 0.0277 mmol). The reaction was stirred at room temperature for 1 h. De-ionized H₂O (56 mL) was added, and stirred for 10 min. The solution was then immediately exchanged with 10 equivalent volumes of 5 mM Phosphate-Citrate buffer (pH 5) using a mPES 30kD 115cm² filter module equipped with a tangential flow filtration system (KrosFlo Research). The solution was then concentrated using the apparatus to 55 mL final volume. A pH value of 5.1 was recorded. Samples were taken for concentration determination by headspace gas chromatography. An aliquot was lyophilized and then reconstituted in 33.3% Acetonitrile-d in Deuterium Oxide at a concentration of 10 mg/mL for ¹H-NMR analysis. Theoretical MW was calculated to be 43,026 g/mol 45,765 g/mol for 006 and 100A respectively.
vii). Using similar techniques, similar amphipathic membrane active polyamines can be readily formed. Particularly, amphipathic membrane active polyamines with molecular weight (Mw) 40-120k protected (25k to 85k deprotected), PDI ranges of 1.03 to 1.2, and monomer ratios of 35% amine monomer/65% hydrophobic group monomer to 70% amine monomer/30% hydrophobic group monomer.

*B) Synthesis of APN 1095-126 (126).*

| MW (protected) | Theoretical MW (deprotected) | PDI | % Amine Incorporation | % Alkyl Incorporation | % End Group Removal | Azides Per Polymer |
|---|---|---|---|---|---|---|
| 66,670 | 47,606 | 1.11 | 56 | 44 | 0 | 4.1 |

Synthesis of APN 1095-126 used dithiobenzoate moiety RAFT agent and AIBN RAFT initiator, compared to the trithiocarbonate moiety RAFT agent and V-65 RAFT initiator used for synthesis of 100A and 006. The conditions for this polymerization required different heating temperatures and times. In addition, this polymer required fractional precipitation. The polymer was not endcapped, but the method of azide addition was the same as 100A and 006.
i). *Materials.* Propyl acrylate was purchased from Polysciences Inc. N-Boc-ethoxyethylamine acrylate was obtained from WuXi Inc. 4-Cyano-4-(phenylcarbonothioylthio) pentanoic acid (CPCPA, RAFT Agent), 2,2'-Azobis(2-methylpropionitrile) (AIBN, radical initiator), Pentafluorophenol, N,N'-Dicyclohexylcarbodiimide and N,N-diisopropylethylamine were purchased from Sigma Aldrich. O-(2-Aminoethyl)-O'-(2-azidoethyl)triethylene Glycol (azido-PEG₄-amine) was purchased from Biomatrik Inc.
ii) *RAFT copolymer of N-Boc-ethoxyethylamine acrylate and propyl acrylate (EAP).* The following procedure was repeated 8 times to yield a total of 4.5513 g fractionated EAP copolymer. Solutions of AIBN (1.035 mg/mL) and RAFT agent CPCPA (50.54 mg/mL) in butyl acetate were prepared. Monomer molar feed was 52% N-Boc-ethoxyethylamine acrylate, 48% propyl acrylate. Theoretical Mw was 75,000. RAFT agent (CPCPA) to initiator (AIBN) molar ratio was 6.67:1.
   N-Boc-ethoxyethylamine acrylate (1.7879 g, 6.9 mmol), propyl acrylate (0.774 mL, 0.7121 g, 6.24 mmol), CPCPA solution (0.184 mL, 0.0333 mmol), AIBN solution (0.793 mL, 0.005 mmol), and butyl acetate (11.02 mL) were added to a 20 mL glass vial with a stir bar. The vial was sealed with a septa cap and the solution bubbled with nitrogen using a long needle with a second needle as the outlet for 1 h. The needles were removed and the vial was heated to 50°C for 24 h with stirring. The solution was allowed to cool to room temperature and transferred to a 50 mL centrifuge tube before hexane (35 mL) was added. The solution was centrifuged for 2 min at 4400 rpm. The supernatant layer was carefully decanted and the bottom layer rinsed with hexane. The bottom layer of each tube was then re-dissolved in dichloromethane (7 mL), precipitated in hexane (40 mL) and centrifuged once more. The supernatant was decanted and the bottom layer rinsed with Hexane before the polymer was dried under reduced pressure for several hours. The yield of crude EAP copolymer was 1.734 g. Samples of the crude copolymer were taken for multi-angle light scattering (MALS), and ¹H-NMR. The dried, crude copolymer was dissolved in DCM (100 mg/mL). Hexane was added until just after the cloud point was reached. The resulting milky solution was centrifuged. The bottom layer was extracted and fully precipitated into hexane. The fraction was centrifuged, after which the copolymer was isolated and dried under vacuum. The yield of isolated fraction of EAP copolymer was 0.602 g. Samples of the fractionated copolymer were taken for ¹H-NMR and MALS. The composition determined by ¹H-NMR was 56% N-Boc-ethoxyethylamine acrylate and 44% propyl acrylate. The Mw determined by MALS was 62,010 g/mol with a polydispersity index (PDI) of 1.14.
iii). *Pentafluorophenol activation of α-end group.* EAP polymer (2 g, 0.0347 mmol), pentafluorophenol (63.8 mg, 0.3466 mmol), N,N'-Dicyclohexylcarbodiimide (71.5 mg, 0.3466 mmol), and dichloromethane (40mL) were added to a 100 mL round bottom flask with a stir bar. The flask was stoppered with a rubber septum and the system was purged with nitrogen for 15 min. The solution was stirred for 16h at room temperature. Additional Pentafluorophenol (63.8 mg, 0.3466 mmol) and N,N'-Dicyclohexylcarbodiimide (71.5 mg, 0.3466 mmol) were added, the flask stoppered with a rubber septum, and the system was purged with nitrogen for 15min. The solution was stirred for 3h at room temperature. The polymer was precipitated with hexane (∼10× volume), centrifuged, and the solvent was decanted. The polymer was dissolved in minimal dichloromethane, precipitated with hexane (∼10× volume), centrifuged, and the solvent was decanted. The polymer was dissolved in minimal ethyl acetate, precipitated with hexane (∼10× volume), centrifuged, and the solvent was decanted. The polymer precipitate was dried under high vacuum until the solid reached a constant weight.
iv). *Azide functionalization of α-end group.* In a 100 ml round bottom flask equipped with a rubber septum and stir bar, polymer from the previous step (1.9g, 0.0329 mmol) was dissolved in dichloromethane (38 mL). Azido-PEG₄-Amine (86.4 mg, 0.3293 mmol) and N,N-Diisopropyl-ethylamine (46.8 mg, 63.1 µL, 0.3622 mmol) were added to the flask with stirring. The system was purged with nitrogen for 15min, and the reaction was left to stir at room temperature overnight. Additional Azido PEG₄ Amine (86.4 mg, 0.3293 mmol) and N,N-Diisopropyl-ethylamine (46.8 mg, 63.1 µL, 0.3622 mmol) were added to the flask, the system was purged with N₂ gas, and the reaction was stirred for 3h at room temperature. The polymer was precipitated with hexane (∼10× volume), centrifuged, and the solvent was decanted. The polymer was dissolved in minimal dichloromethane, precipitated with hexane (∼10× volume), centrifuged, and the solvent was decanted. The polymer precipitate was dried under high vacuum until the solid reached a constant weight. The yield of Azide functionalized EAP was 1.77g. Samples of the copolymer were taken for multi-angle light scattering (MALS), and ¹H-NMR. The composition determined by ¹H-NMR was 56% N-Boc-ethoxyethylamine acrylate and 44% propyl acrylate. The Mw determined by MALS was 66,670 g/mol with a polydispersity index (PDI) of 1.11.
v). *Boc Deprotection and Tangential Flow Filtration.* In a 100 mL round bottom flask, 2M HCl in acetic acid (28mL) was added to Azide functionalized EAP copolymer (1.67 g, 0.0277 mmol). The reaction was stirred at room temperature for 1 hour. De-ionized H₂O (56 mL) was added, and stirred for 10 min. The solution was then immediately exchanged with 10 equivalent volumes of 5 mM Phosphate-Citrate buffer (pH 5) using a mPES 30kD 115cm² filter module equipped with a tangential flow filtration system (KrosFlo Research). The solution was then concentrated using the apparatus to 55 mL final volume. A pH value of 5.1 was recorded. Samples were taken for concentration determination by headspace gas chromatography. An aliquot was lyophilized and then reconstituted in 33.3% Acetonitrile-d in Deuterium Oxide at a concentration of 10 mg/mL for ¹H-NMR analysis. Theoretical MW was calculated to be 43,026 g/mol.

C). *RGD-PEGₙ-FCitFP-TFP and PEGₙ-FCitFP-TFP agent synthesis.* The modifying agent precursor (di-Boc)RGD(OtBu)-APBA-PEGₙ-FCitFP-COOH was prepared using general Fmoc chemistry solid phase synthesis using 2-Cl-Trt resin preloaded with Fmoc-Proline-OH. To Resin-Pro-Fmoc was added sequentially (following Fmoc deprotection at each step): FMoc-Phe-OH, Fmoc-Cit-OH, Fmoc-Phe-OH, Fmoc-NH-PEGₙ-COOH, 4-(N-Fmoc-p-aminophenoxy)-butyric acid (APBA), Fmoc-Asp(OtBu)-OH, Fmoc-Gly-OH, and diboc-m-guanidino benzoic acid.
(diboc)RGD(OtBu)-APBA-PEGₙ-FCitFP-COOH (458 mg, 0.200 mmols) and TFP (66.5 mg, 0.400 mmols) were dissolved in anhydrous DCM (5.0 mL) and cooled to 0°C in an ice/water bath while stirring under Argon. EDC (77 mg, 0.400 mmols) was added and the reaction mixture stirred in an ice/water bath at 0°C for 30 min. Reaction progress was monitored by TLC (8.5:1.5 CHCl₃:MeOH) and was complete after 90 min with no starting material observed by TLC. The reaction mixture was diluted to 100 mL total volume with DCM, washed 3 × 40 mL with DI H₂O (pH=5), and washed 1 × 40 mL aqueous saturated NaCl solution. The organics were then dried over Na₂SO₄, and concentrated on a rotovap to yield 448 mg (92 % yield) of a tan/orange foam. The structure was confirmed by ¹H NMR, and ESI MS (Reaction shown above for PEG₂₀ (n = 20)). 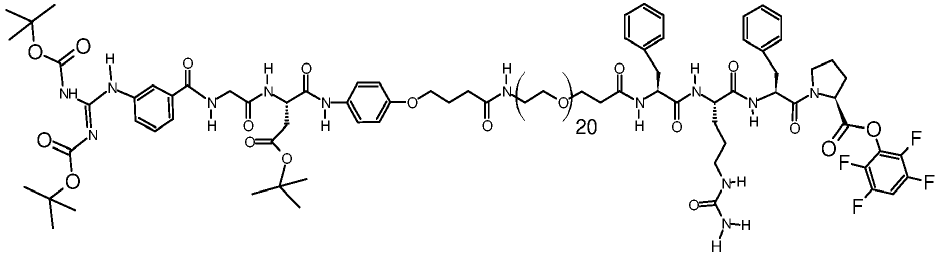 (diboc)RGD(OtBu)-APBA-PEGₙ-FCitFP-TFP (shown for n = 20)
(diboc)RGD(OtBu)-PEGn-FCitFP-TFP (497 mg, 0.204 mmols) was dissolved in [9.25:0.75:0.50] TFA:H₂O:Thioanisole (5.0 mL) and stirred at room temperature in a closed flask for 45 min. Reaction completion was confirmed by MS (ESI, scan neg, 300-3000) with no masses related to starting material or partially deprotected intermediates observed. The reaction mixture was then precipitated into 45 mL diethyl ether, spun down, the supernatant poured off, and washed 2×10 mL diethyl ether and dried on high vacuum overnight. The final product was purified on prep HPLC using a Thermo Aquasil C18 5 um semi prep column, with mobile phases 0.1% TFA in H₂O and ACN. Each injection was 50 mg of crude material dissolved in 3.0 mL of 0.1% TFA in [61:39] H₂O:ACN run on a gradient of (indicated in %B) 39-(5)-39-(35)-43-(5)-95-(10)-95-(2)-39-(5)-39. Each sample for injection was prepared (dissolved) within 15 minutes of being injected and positive fractions were pooled in one flask and kept cold in the freezer until the last injection of the day had finished. The positive fractions were then concentrated on the rotovap with a bath temperature of 32°C to dryness, then chased 2× with ACN/Toluene, then 3× with ACN and then dried on high vacuum overnight. Out of 257 mg injected crude, 180 mg (70%) was isolated as pure material (Reaction shown above for PEG₂₀ (n = 20)). 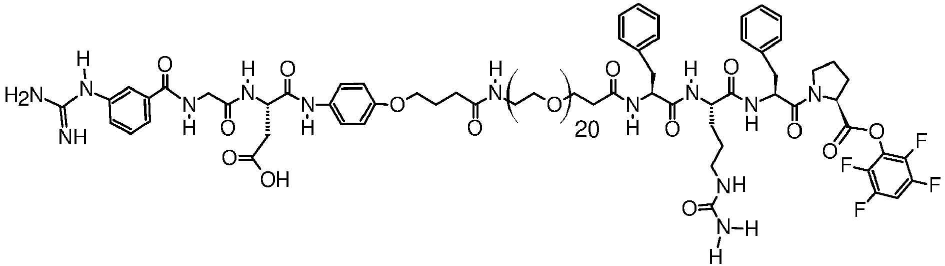 RGD-PEGₙ-FCitFP-TFP (shown for n = 20)
*4-(N-Fmoc-p-aminophenoxy)-butyric acid **I** synthesis.* p-Nitro-phenol (**2**) (7.5 g, 53.9 mmole) was combined with ethyl 4-bromobutyrate (8.45 ml, 59 mmol) and K₂CO₃ (7.5 g, 54 mmole) in DMF (75 mL). The mixture was stirred for 2 h at 100°C. DMF was removed and the crude product was diluted in a mixture of 3:1 mixture of 2 N NaOH and methanol and stirred 4 h at RT. The reaction mixture was acidified with 6 M HCl. The white precipitate was collected to yield 4-(p-Nitrophenyloxy)-butyric acid **3:** (10.9 g, 90% yield).
4-(p-Nitrophenyloxy)-butyric acid **3** (37.1 g, 165 mmole) was dissolved in MeOH (1 L) with ammonium formate (35 g, 555 mmole) and 10% Pd/C (Degussa Type) (3.5 g) was added. The mixture was refluxed at 65°C overnight. The reaction was filtered with celite to yield a reddish brown solid of product 4-(p-Aminophenyloxy)-butyric acid **4** (30.5 g, 95 % yield).
4-(p-Aminophenyloxy)-butyric acid **4** (5.1 g, 26 mmole) was dissolved in 6:4 a mixture of an aqueous saturated NaHCO₃ (3.36 g, 40 mmol) in H₂O (450 mL) and THF (300 ml) to make a white slurry. Fmoc-OSu (8.82 g, 26.1 mmole) was added and the reaction was stirred for 4 h. The acetone was removed, the reaction was acidified (HCl), and the off-white precipitate was collected and triturated in 1N HCl to yield 9.6 g of product 4-(N-Fmoc-p-aminophenoxy)-butyric acid **1** (88% yield). 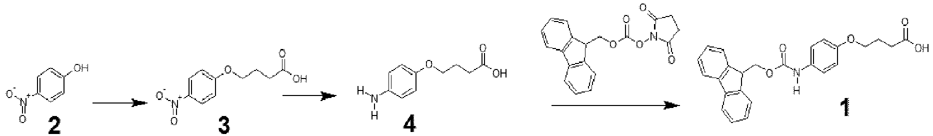
*diBoc-m-guanidino-benzoic acid* **5** was synthesized according to Riches AG et al. Tetrahedron (2012) 68, p. 9448-9455.
PEGₙ-FCitFP modifying agents were made using similar chemistry.
D) *Masking (modification) of polymer* The mono azide-polymer was reacted with protease cleavable-RGD agent (RGD-PEG₈-ACit-PNP, RDG-PEG₈-FCitFP-TFP, RGD-PEG₁₅-FCitFP-TFP, RGD-PEG₁₉-FCitFP-TFP, or RGD-PEG₂₀-FCitFP-TFP) at weight ratios of 1:0.125, 1:0.25, 1:0.5, 1:1, 1:1.5, 1:2 (polymer:RGD) in 50 mM HEPES, pH 8.5 buffer for 4 h at RT. The modified polymer was then reacted with protease cleavable-PEG agent (PEG₆-ACit-PABC-PNP, PEG₁₂-ACit-PABC-PNP, PEG₁₂-FCit-PABC-PNP, PEG₁₂-FCitFP-TFP) at a weight ratio of 1:8 (polymer:PEG) in 50 mM HEPES, pH 8.5 buffer for 2 h at RT. Alkyne-RNAi trigger at a weight ratio of 1:0.3 (polymer:Alkyne-RNAi trigger) was added to the modified polymer in 100 mM sodium acetate-acetic acid buffer solution, pH 5.0 for 5 days at RT. The completed conjugate was TFF purified and conjugation efficiency determined.
E) *Evaluation of in vivo delivery using tetrapeptide modified DPC delivery polymers.* Kidney RCC tumor-bearing mice were treated with a single tail vein injection of isotonic glucose (G1) or the indicated Hif2α-ITG-DPC (Hif2α-ITG-DPC = Hif2α RNAi trigger-delivery polymer conjugate. The delivery polymer is modified with RGD ligand and PEG modifying agents). Mice were euthanized 72 h after injection and total RNA was prepared from kidney tumor using Trizol reagent following manufacture's recommendation. Relative HiF2α mRNA levels were determined by RT-qPCR as described below and compared to mice treated with delivery buffer (isotonic glucose) only (Table 8).

**Table. 8**

| HiF2α RNAi trigger | | µg polymer | RGD agent | | | PEG agent | Rel Exp day 4 | | |
|---|---|---|---|---|---|---|---|---|---|
| ID | µg | | identity | modif. level | RGD/ polymer | | ave. | low error | high error |
| - | | | | | | | 1.000 | 0.060 | 0.064 |
| AD01031 | 80 | 280 | RGD-PEG8-HyNic | | | PEG12-AC-PABC | 0.300 | 0.074 | 0.098 |
| AD01031 | 80 | 280 | RGD-PEG8-AC-PABC | 0.4× | | PEG6-AC-PABC | 0.311 | 0.055 | 0.067 |
| AD01214 | 80 | 280 | RGD-PEG8-HyNic | | | PEG12-FC-PABC | 0.489 | 0.022 | 0.023 |
| AD01214 | 80 | 280 | RGD-PEG8-HyNic | | | PEG12-FCFP-TFP | 0.210 | 0.032 | 0.038 |
| AD01214 | 80 | 280 | RGD-PEG-AC-PABC | | | PEG6-AC-PABC | 0.360 | 0.019 | 0.021 |
| AD01214 | 80 | 280 | RGD-PEG8-ACFP | 1× | | PEG6-AC-PABC | 0.288 | 0.005 | 0.005 |
| AD01214 | 115 | 375 | RGD-PEG8-HyNic | | | PEG12-FCFP-TFP | 0.258 | 0.033 | 0.038 |
| AD01214 | 112.5 | 375 | RGD-PEG15-FCFP-TFP | 0.5× | 10.8 | PEG12-AC-PABC | 0.193 | 0.046 | 0.061 |
| AD01214 | 112.5 | 375 | RGD-PEG15-FCFP-TFP | 1× | 16.1 | PEG12-AC-PABC | 0.182 | 0.007 | 0.008 |
| AD01214 | 112.5 | 375 | RGD-PEG15-FCFP-TFP | 2× | 29.0 | PEG12-AC-PABC | 0.182 | 0.031 | 0.038 |
| AD01214 | 112.5 | 375 | RGD-PEG19-FCFP-TFP | 0.5× | 10.7 | PEG12-AC-PABC | 0.163 | 0.023 | 0.027 |
| AD01214 | 112.5 | 375 | RGD-PEG19-FCFP-TFP | 1× | 18.5 | PEG12-AC-PABC | 0.114 | 0.011 | 0.012 |
| AD01214 | 112.5 | 375 | RGD-PEG19-FCFP-TFP | 2× | 31.1 | PEG12-AC-PABC | 0.182 | 0.047 | 0.063 |
| AD01214 | 112.5 | 375 | RGD-PEG15-FCFP-TFP | 1× | | PEG12-FCFP-TFP | 0.148 | 0.079 | 0.169 |
| AD01214 | 112.5 | 375 | RGD-PEG19-FCFP-TFP | 1× | | PEG12-FCFP-TFP | 0.188 | 0.026 | 0.030 |
| AD01214 | 112.5 | 300 | RGD-PEG8-HyNic | | | PEG12-AC-PABC | 0.357 | 0.069 | 0.086 |
| AD01214 | 112.5 | 375 | RGD-PEG20-FCFP-TFP | 0.125× | 1.9 | PEG12-AC-PABC | 0.169 | 0.052 | 0.075 |
| AD01214 | 112.5 | 375 | RGD-PEG20-FCFP-TFP | 0.25× | 3.4 | PEG12-AC-PABC | 0.168 | 0.029 | 0.035 |
| AD01214 | 112.5 | 375 | RGD-PEG20-FCFP-TFP | 0.5× | 6.6 | PEG12-AC-PABC | 0.130 | 0.004 | 0.005 |
| AD01214 | 112.5 | 375 | RGD-PEG20-FCFP-TFP | 1× | 13 | PEG12-AC-PABC | 0.121 | 0.016 | 0.018 |
| AD01214 | 112.5 | 375 | RGD-PEG20-FCFP-TFP | 1.5× | 20 | PEG12-AC-PABC | 0.135 | 0.018 | 0.020 |

Example 9. Additional structures illustrating utility of the described tetrapeptide linkers. 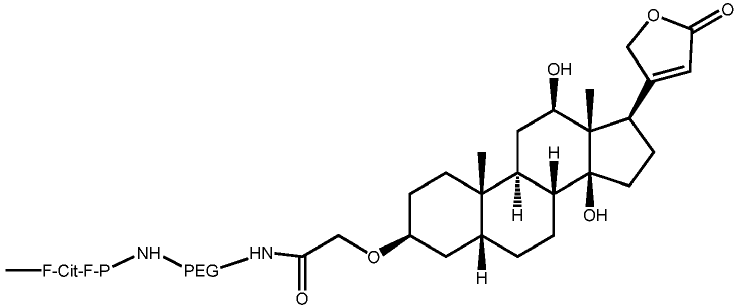 R⁵-FCitFP-Digoxin (drug molecule) 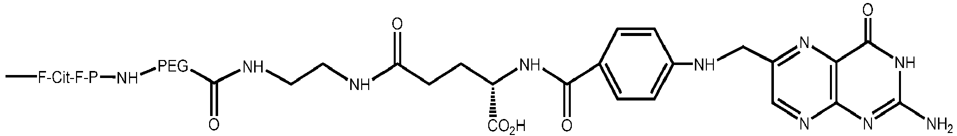 R⁵-FCitFP-Folate (cell receptor ligand) 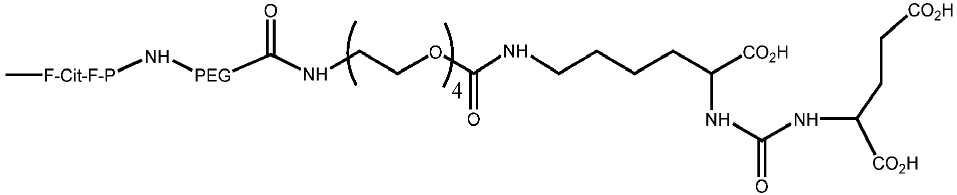 R⁵-FCitFP-PSMA 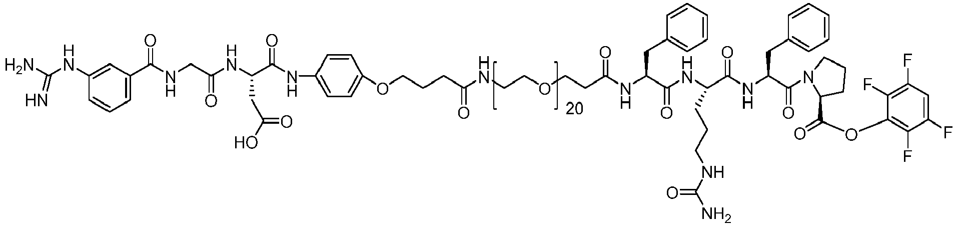 RGD-FCitFP-TFT reactive group (cell receptor (RGD mimic-containing modifying agent)

## Claims

1. A tetrapeptide linker comprising the structure represented by:

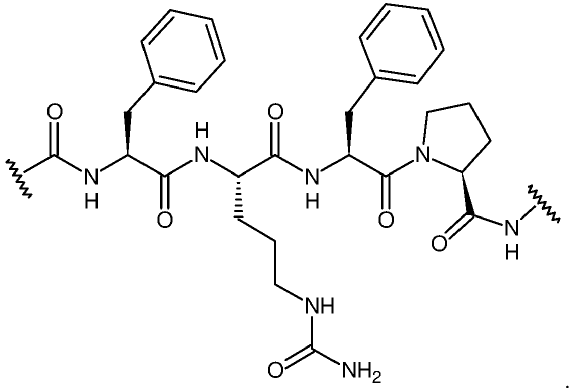

2. A composition comprising:
R⁵-A⁴-A³-A²-A¹-R⁶ (formula IV)
wherein
R⁵ is a first compound,
R⁶ is an amine-containing second compound, preferably a polyamine, more preferably a membrane active polyamine;
A⁴ is L-phenylalanine;
A³ is L-citrulline;
A² is L-phenylalanine;
A¹ is L-proline, and
A¹ is linked to R⁶ via an amide bond.

3. Composition comprising:
(R⁵-A⁴-A³-A²-A¹)ₙ-P
wherein
R⁵ comprises a targeting group or a steric stabilizer;
A⁴ is L-phenylalanine;
A³ is L-citrulline;
A² is L-phenylalanine;
A¹ is L-proline,
P comprises a polyamine, preferably a membrane active polyamine;
n is an integer greater than or equal to 1, and
each A¹ is linked to an amine on the polyamine via an amide bond.

4. The composition of claim 3 wherein n is greater than 50%, greater than 60%, greater than 70%, greater than 75%, greater than 80%, greater than 85%, greater than 90%, or greater than 95% of the number of amines on the polyamine.

5. A composition comprising:
(R⁵-A⁴-A³-A²-A¹)ₙ-P-(A^{1'}-A^{2'}-A^{3'}-A^{4'}-R⁸)ₘ
wherein
A⁴ is L-phenylalanine,
A³ is L-citrulline,
A² is L-phenylalanine,
A¹ is L-proline
A^{4'} is L-phenylalanine,
A^{3'} is L-citrulline,
A^{2'} is L-phenylalanine,
A^{1'} is L-proline,
R⁵ comprises a targeting group,
R⁸ comprises a steric stabilizer,
P comprises a polyamine, preferably a membrane active polyamine;
n and m are each integers greater than or equal to 1, and
each A¹ and A^{1'} are linked to amines on the polyamine via amide bonds.

6. The composition of claim 5 wherein the value of n+m is greater than 50%, greater than 60%, greater than 70%, greater than 75%, greater than 80%, greater than 85%, greater than 90%, or greater than 95% of the number of amines on the polyamine.

7. A tetrapeptide modifying agent for reversibly attaching a first compound to an amine-containing compound via a biologically labile tetrapeptide linker, wherein the tetrapeptide modifying agent has the structure represented by: 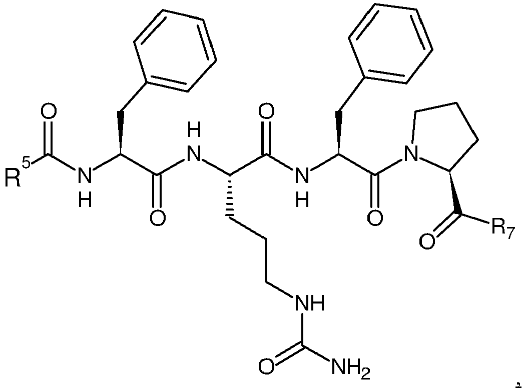 wherein
R⁵ represents the first compound, and
R⁷ comprises an amine-reactive group.

## Patentansprüche

1. Tetrapeptid-Linker, der die Struktur aufweist, die dargestellt wird durch:

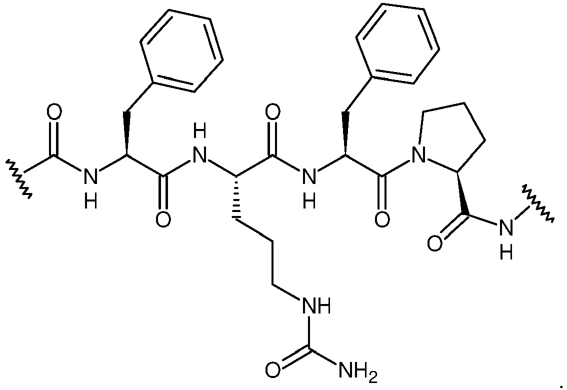

2. Zusammensetzung, die aufweist:
R⁵-A⁴-A³-A²-A¹-R⁶ (Formel IV)
wobei
R⁵ eine erste Verbindung ist,
R⁶ eine Amin-enthaltende zweite Verbindung ist, vorzugsweise ein Polyamin, bevorzugter ein membranaktives Polyamin;
A⁴ L-Phenylalanin ist;
A³ L-Citrullin ist;
A² L-Phenylalanin ist;
A¹ L-Prolin ist, und
A¹ an R⁶ über eine Amidbindung gebunden ist.

3. Zusammensetzung, die aufweist:
(R⁵-A⁴-A³-A²-A¹)ₙ-P
wobei
R⁵ eine Zielgruppe oder einen sterischen Stabilisator aufweist;
A⁴ L-Phenylalanin ist;
A³ L-Citrullin ist;
A² L-Phenylalanin ist;
A¹ L-Prolin ist,
P ein Polyamin, vorzugsweise ein membranaktives Polyamin aufweist;
n eine Ganzzahl größer als oder gleich 1 ist, und
jedes A¹ an einem Amin am Polyamin über eine Amidbindung gebunden ist.

4. Zusammensetzung nach Anspruch 3, wobei n größer als 50 %, größer als 60 %, größer als 70 %, größer als 75 %, größer als 80 %, größer als 85 %, größer als 90 % oder größer als 95 % der Anzahl der Amine am Polyamin ist.

5. Zusammensetzung, die aufweist:
(R⁵-A⁴-A³-A²-A¹)ₙ-P-(A^{1'}-A^{2'}-A^{3'}-A^{4'}-R⁸)ₘ
wobei
A⁴ L-Phenylalanin ist;
A³ L-Citrullin ist;
A² L-Phenylalanin ist;
A¹ L-Prolin ist,
A^{4'} L-Phenylalanin ist;
A^{3'} L-Citrullin ist;
A^{2'} L-Phenylalanin ist;
A^{1'} L-Prolin ist,
R⁵ eine Zielgruppe aufweist;
R⁸ einen sterischen Stabilisator aufweist;
P ein Polyamin, vorzugsweise ein membranaktives Polyamin aufweist;
n und m Ganzzahlen größer als oder gleich 1 sind, und
ein jedes A¹ und A^{1'} an Aminen am Polyamin über Amidbindungen gebunden ist.

6. Zusammensetzung nach Anspruch 5, wobei der Wert n + m größer als 50 %, größer als 60 %, größer als 70 %, größer als 75 %, größer als 80 %, größer als 85 %, größer als 90 % oder größer als 95 % der Anzahl der Amine am Polyamin ist.

7. Tetrapeptid-Modifikationsmittel, um eine erste Zusammensetzung an einer Aminenthaltenden Zusammensetzung über einen biologisch labilen Tetrapeptid-Linker reversibel anzubinden, wobei das Tetrapeptid-Modifizierungsmittel die Struktur aufweist, die dargestellt wird durch: 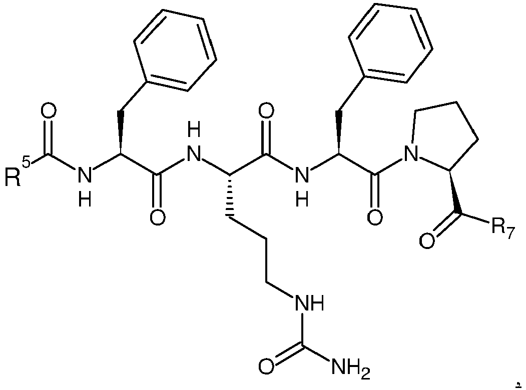 wobei
R⁵ die erste Verbindung darstellt, und
R⁷ eine aminoreaktive Gruppe aufweist.

## Revendications

1. Agent de liaison tétrapeptidique comprenant la structure représentée par :

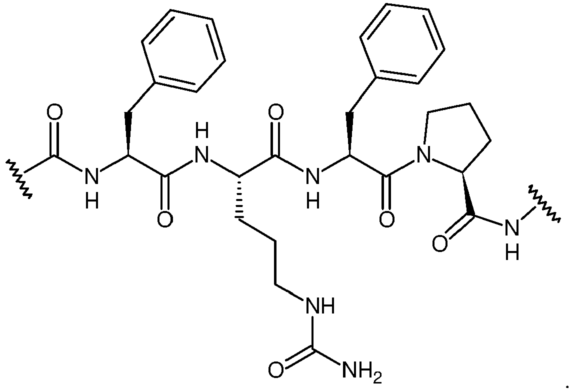

2. Composition comprenant :
R⁵-A⁴-A³-A²-A¹-R⁶ (formule IV)
dans laquelle
R⁵ est un premier composé,
R⁶ est un second composé contenant une amine, de préférence une polyamine, de manière davantage préférée une polyamine active sur une membrane ;
A⁴ est une L-phénylalanine ;
A³ est une L-citrulline ;
A² est une L-phénylalanine ;
A¹ est une L-proline, et
A¹ est lié à R⁶ via une liaison amide.

3. Composition comprenant :
(R⁵-A⁴-A³-A²-A¹)ₙ-P
dans laquelle
R⁵ comprend un groupe de ciblage ou un stabilisant stérique ;
A⁴ est une L-phénylalanine ;
A³ est une L-citrulline ;
A² est une L-phénylalanine ;
A¹ est une L-proline,
P comprend une polyamine, de préférence une polyamine active sur une membrane ;
n est un nombre entier supérieur ou égal à 1, et
chaque A¹ est lié à une amine sur la polyamine via une liaison amide.

4. Composition selon la revendication 3 dans laquelle n est supérieur à 50 %, supérieur à 60 %, supérieur à 70 %, supérieur à 75 %, supérieur à 80 %, supérieur à 85 %, supérieur à 90 %, ou supérieur à 95 % du nombre d'amines de la polyamine.

5. Composition comprenant :
(R⁵-A⁴-A³-A²-A¹)ₙ-P-(A^{1'}-A^{2'}-A^{3'}-A^{4'}-R⁸)ₘ
dans laquelle
A⁴ est une L-phénylalanine,
A³ est une L-citrulline,
A² est une L-phénylalanine,
A¹ est une L-proline
A^{4'} est une L-phénylalanine,
A^{3'} est une L-citrulline,
A^{2'} est une L-phénylalanine,
A^{1'} est une L-proline,
R⁵ comprend un groupe de ciblage,
R⁸ comprend un stabilisant stérique,
P comprend une polyamine, de préférence une polyamine active sur une membrane ;
n et m sont chacun des nombres entiers supérieurs ou égaux à 1, et
chaque A¹ et A^{1'} sont liés à des amines sur la polyamine via des liaisons amide.

6. Composition selon la revendication 5 dans laquelle la valeur de n + m est supérieure à 50 %, supérieure à 60 %, supérieure à 70 %, supérieure à 75 %, supérieure à 80 %, supérieure à 85 %, supérieure à 90 %, ou supérieure à 95 % du nombre d'amines de la polyamine.

7. Agent de modification tétrapeptidique pour fixer de manière réversible un premier composé à un composé contenant une amine via un agent de liaison tétrapeptidique biologiquement labile, dans lequel l'agent de modification tétrapeptidique présente la structure représentée par : 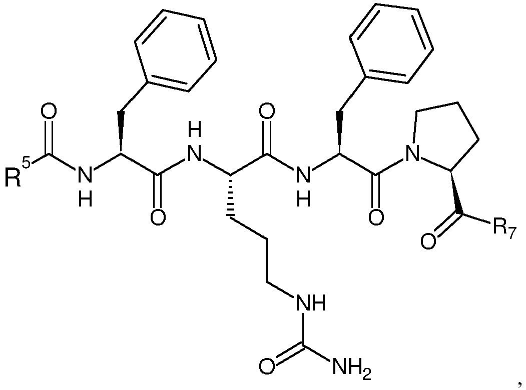 dans laquelle
R⁵ représente le premier composé, et
R⁷ comprend un groupe réactif avec les amines.


### SEQUENCE LISTING

<110> Arrowhead Pharmaceuticals, Inc. Carlson, Jeffrey C Blokhin, Andrei V
<120> Biologically Cleavable Tetrapeptide Linking Agents
<130> 30630-WO1
<150> 62168244
   <151> 2015-05-29
<150> 62235833
   <151> 2015-10-01
<160> 19
<170> PatentIn version 3.5
<210> 1
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<400> 1 
Ala Xaa Ala Ala
1
<210> 2
   <211> 4
   <212> PRT
   <213> Artificial Sequence
<220>
   <223> tetrapeptide
<220>
   <221> MISC_FEATURE
   <222> (2)..(2)
   <223> Xaa = citrulline
<400> 2 
Ala Xaa Ala Pro
1
<210> 3
   <211> 4
   <212> PRT
   <213> Artificial Sequence
<220>
   <223> tetrapeptide
<220>
   <221> MISC_FEATURE
   <222> (2)..(2)
   <223> Xaa = citrulline
<400> 3 
Ala Xaa Phe Pro
1
<210> 4
   <211> 20
   <212> DNA
   <213> Artificial Sequence
<220>
   <223> Factor 7 siRNA sense strand
<220>
   <221> modified_base
   <222> (1)..(19)
   <223> ribonucleotide
<400> 4
   gcaaaggcgu gccaacucat 20
<210> 5
   <211> 4
   <212> PRT
   <213> Artificial Sequence
<220>
   <223> tetrapeptide
<220>
   <221> MISC_FEATURE
   <222> (1)..(1)
   <223> Xaa = Citrulline
<400> 5 
Xaa Ala Ala Ala
1
<210> 6
   <211> 21
   <212> DNA
   <213> Artificial Sequence
<220>
   <223> Factor 7 siRNA antisense strand
<220>
   <221> modified_base
   <222> (2)..(19)
   <223> ribonucleotide
<400> 6
   tgaguuggca cgccuuugct t 21
<210> 7
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<400> 7 
Phe Xaa Ala Ala
1
<210> 8
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<400> 8 
Phe Xaa Ala Pro
1
<210> 9
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<220>
   <221> MISC_FEATURE
   <222> (4)..(4)
   <223> Xaa = N-methyl alanine
<400> 9 
Phe Xaa Phe Xaa
1
<210> 10
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<400> 10 
Phe Xaa Phe Ala
1
<210> 11
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<400> 11 
Phe Xaa Phe Leu
1
<210> 12
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<400> 12 
Phe Xaa Phe Pro
1
<210> 13
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<400> 13 
Phe Xaa Val Pro
1
<210> 14
   <211> 4
   <212> PRT
   <213> Artificial Sequence
<220>
   <223> tetrapeptide
<400> 14 
Phe Asp Phe Pro
1
<210> 15
   <211> 4
   <212> PRT
   <213> Artificial Sequence
<220>
   <223> tetrapeptide
<400> 15 
Phe Lys Phe Leu
1
<210> 16
   <211> 4
   <212> PRT
   <213> Artificial Sequence
<220>
   <223> tetrapeptide
<400> 16 
Phe Lys Phe Pro
1
<210> 17
   <211> 4
   <212> PRT
   <213> Artificial Sequence
<220>
   <223> tetrapeptide
<400> 17 
Phe Asn Phe Pro
1
<210> 18
   <211> 4
   <212> PRT
   <213> Artificial Sequence
<220>
   <223> tetrapeptide
<400> 18 
Gly Phe Leu Gly
1
<210> 19
   <211> 4
   <212> PRT
   <213> Artificial Sequence
<220>
   <223> tetrapeptide
<220>
   <221> MISC_FEATURE
   <222> (2)..(2)
   <223> Xaa = Citrulline
<400> 19

Val Xaa Phe Pro
1